(19) Europäisches Patentamt / European Patent Office / Office européen des brevets

(11) EP 4 722 389 A1

(12) EUROPEAN PATENT APPLICATION

(43) Date of publication:
08.04.2026 Bulletin 2026/15

(21) Application number: 24204475.8

(22) Date of filing: 03.10.2024

(51) International Patent Classification (IPC):
*C12Q 1/6886* (2018.01)

(52) Cooperative Patent Classification (CPC):
**C12Q 1/6886;** C12Q 2600/106; C12Q 2600/118;
C12Q 2600/158

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventor: **HOFFMANN, Ralf Dieter**
**Eindhoven (NL)**

(74) Representative: **Philips Intellectual Property &
Standards**
**High Tech Campus 52**
**5656 AG Eindhoven (NL)**

Remarks:
The complete document including Reference
Table(s) and the Sequence Listing(s) can be
downloaded from the EPO website

(54) **PREDICTION OF AN OUTCOME OF A SUBJECT SUFFERING FROM A MELANOMA**

(57) The invention relates to a method of predicting an outcome of a subject having a melanoma based on the expression level of one or more genes. The invention further describes methods for determining an optimal treatment strategy based on gene expression levels. In addition the invention provides an immune checkpoint inhibitor for use in the treatment of melanoma, the use comprising determining the outcome base on gene expression levels and providing the immune checkpoint inhibitor when a favorable response to treatment is predicted.

Fig. 20

CD4

HR = 0.29 (0.19 - 0.45)
logrank P = 2.4e-09

Expression
— low
— high

| | Number at risk | | | | |
|---|---|---|---|---|---|
| low | 54 | 22 | 2 | 0 | 0 |
| high | 144 | 79 | 11 | 1 | 0 |

EP 4 722 389 A1

**Description**

FIELD OF THE INVENTION

The invention relates to a method of predicting an outcome of a subject suffering from a melanoma, and to an apparatus for predicting an outcome of a subject suffering from a melanoma. Moreover, the invention relates to a diagnostic kit, to a use of the kit, to a use of the kit in a method of predicting an outcome of a subject suffering from a melanoma, to a use of a gene expression profile in a method of predicting an outcome of a subject suffering from a melanoma, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumors, which are self-limited and do not invade or metastasize.

Melanoma is the most aggressive type of skin cancer; it develops from the melanin-producing cells known as melanocytes. It typically occurs in the skin, but may rarely occur in the mouth, intestines, or eye. The primary cause of melanoma is ultraviolet light (UV) exposure in those with low levels of the skin pigment melanin. Alternatively mutations in the MCR1, CDKN2A, p53 or CDK4 genes, among others, can greatly increase melanoma susceptibility. Melanoma is most commonly diagnosed by visually inspecting the area. Following a visual examination and a dermatoscopic exam, or in vivo diagnostic tools such as a confocal microscope, confirmation of the clinical diagnosis is typically done with a skin biopsy. A skin biopsy performed under local anesthesia is often required to assist in making or confirming the diagnosis and in defining severity. The tumor may also be removed by elliptical excisional biopsies, followed by histological analysis and Breslow scoring.

Treatment of melanoma typically involves excisional biopsies. Complete surgical excision with adequate surgical margins and assessment for the presence of detectable metastatic disease along with short- and long-term followup is standard. Melanomas that spread usually do so to the lymph nodes in the area of the tumor before spreading elsewhere. Attempts to improve survival by removing lymph nodes surgically (lymphadenectomy) were associated with many complications, but no overall survival benefit. Adjuvant treatment after surgery may reduce the risk of recurrence after surgery, especially in high-risk melanomas. The most common adjuvant treatment is immune checkpoint inhibitor (ICI) treatment for up to a year post-surgery. Although some patients benefit from ICI treatment, not all patients respond to ICI treatment. Furthermore ICI treatment is expensive, therefore reliable tests are needed to reliably test the outcome of a melanoma patient and more particularly the effectiveness of ICI treatment.

In conclusion, an improved understanding of the (molecular) mechanisms of melanoma, and the emerging availability of treatments underlines the strong need for better prediction of response to treatment remains. As many articles have concluded, there is a strong need for biomarkers that aid in diagnosis and treatment, that identify recurrent disease, and that can indicate treatment response. This unmet need is provided by the methods and products as defined in the appended claims.

SUMMARY OF THE INVENTION

The inventors herein describe gene signatures that can be used to predict an outcome for melanoma patients. In addition the inventors describe that patients with a poor predicted outcome benefit from treatment with Pembroluzimab.

Therefore in a first aspect, the invention relates to Pembroluzimab or an equivalent thereof for use in the treatment of a melanoma in a subject, the use comprising: determining or receiving the result of a determination of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and determining the prediction of the outcome based on one or more gene expression levels, wherein the outcome is a poor outcome or a good outcome, and when the predicted outcome is a poor outcome Pembroluzimab or an equivalent thereof is administered to the subject.

In a second aspect the invention relates to a method of predicting an outcome for a subject having a melanoma, the method comprising: determining or receiving the result of a determination of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and determining the prediction of the outcome based on one or more gene expression levels, wherein the outcome is a poor outcome or a good outcome.

In a third aspect the invention relates to an apparatus for predicting an outcome for a subject having a melanoma, comprising: an input adapted to receive data indicative of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and a processor adapted to determine the prediction of the outcome based on the one or more gene expression levels, wherein the outcome is a poor outcome or a good outcome.

In a fourth aspect the invention relates to a computer program product comprising instructions which, when the program is executed by a computer, cause the computer to carry out a method for predicting an outcome for a subject having a melanoma comprising: receiving data indicative of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and determining a prediction of the outcome based on the one or more gene expression levels wherein the outcome is a poor outcome or a good outcome.

In a fifth aspect the invention relates to the use of a kit for predicting an outcome for a subject having a melanoma, the use comprising: determining one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1 in a sample obtained from a subject having a melanoma; and predicting the outcome based on the one or more gene expression levels wherein the outcome is a poor outcome or a good outcome, wherein the kit comprises means for determining one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1.

BRIEF DESCRIPTION OF THE DRAWINGS

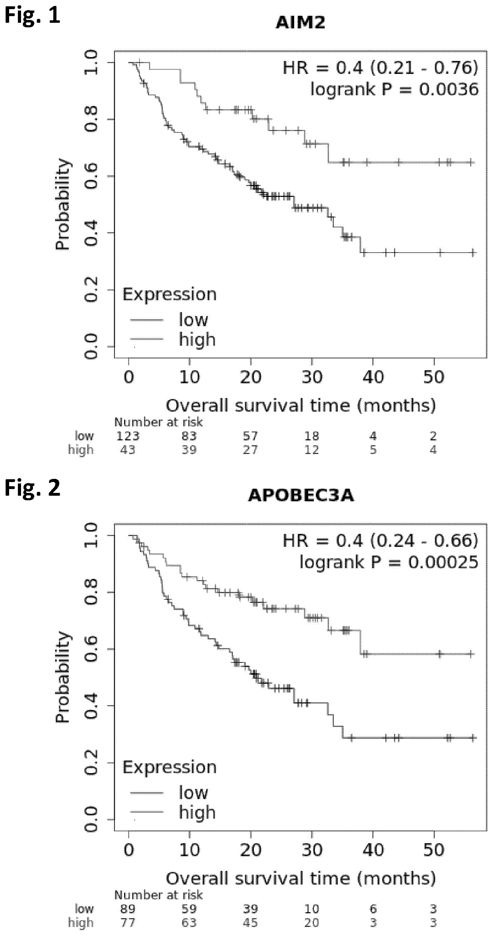

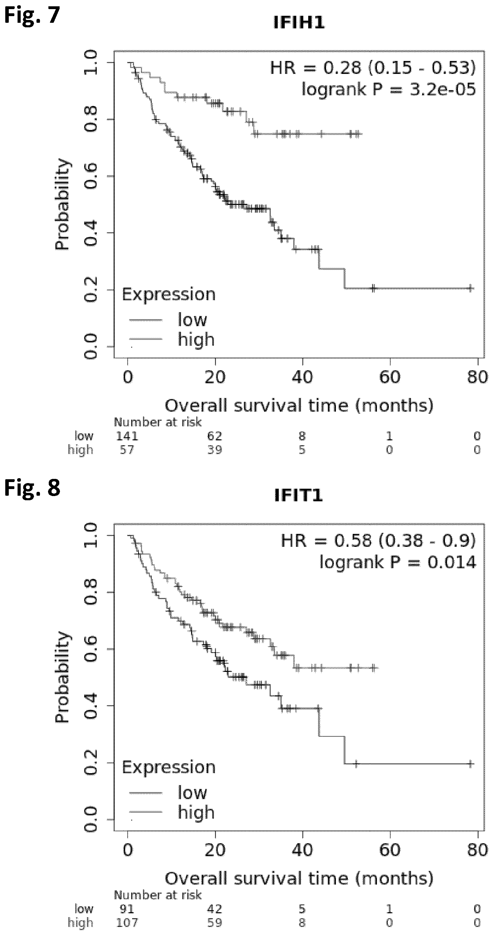

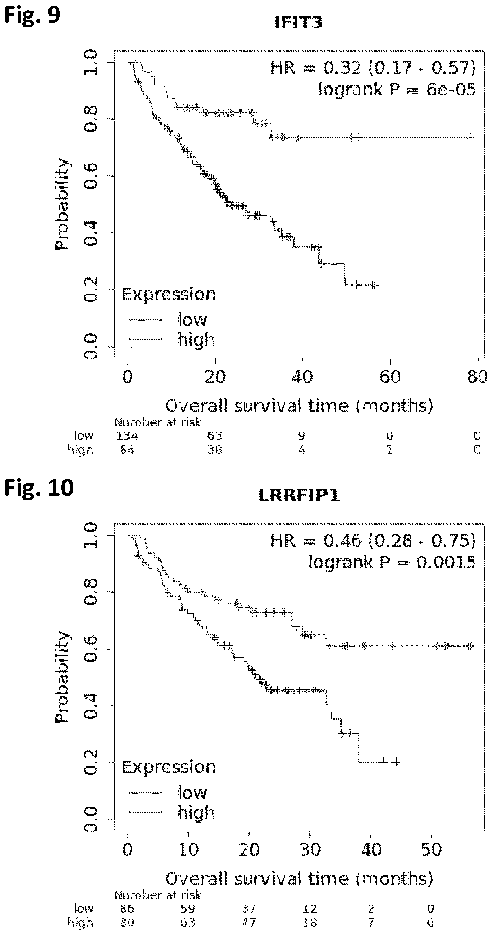

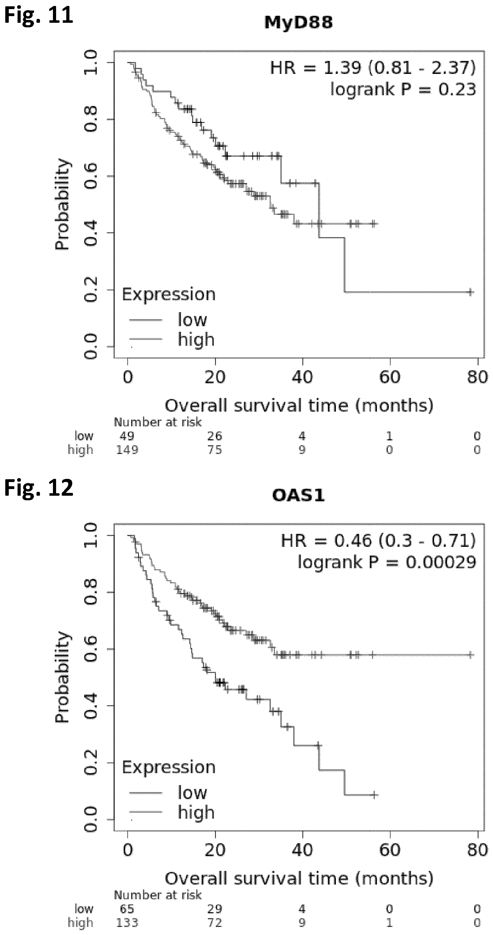

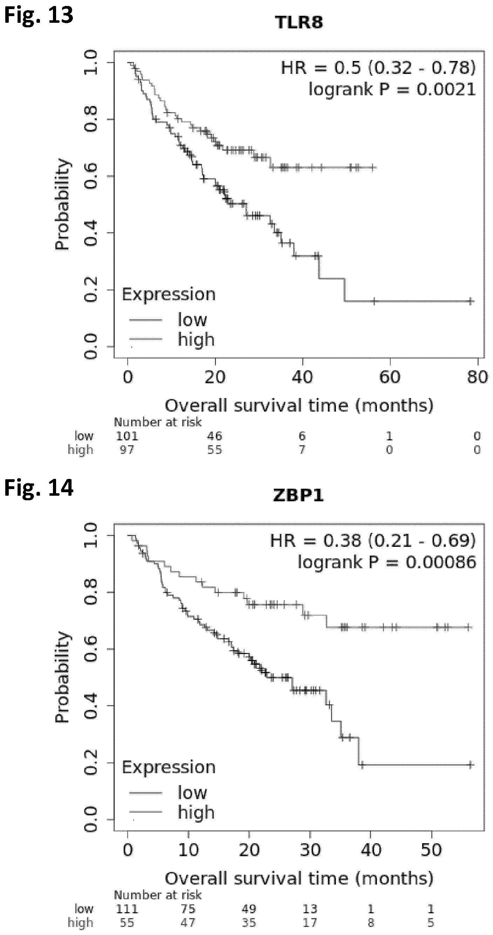

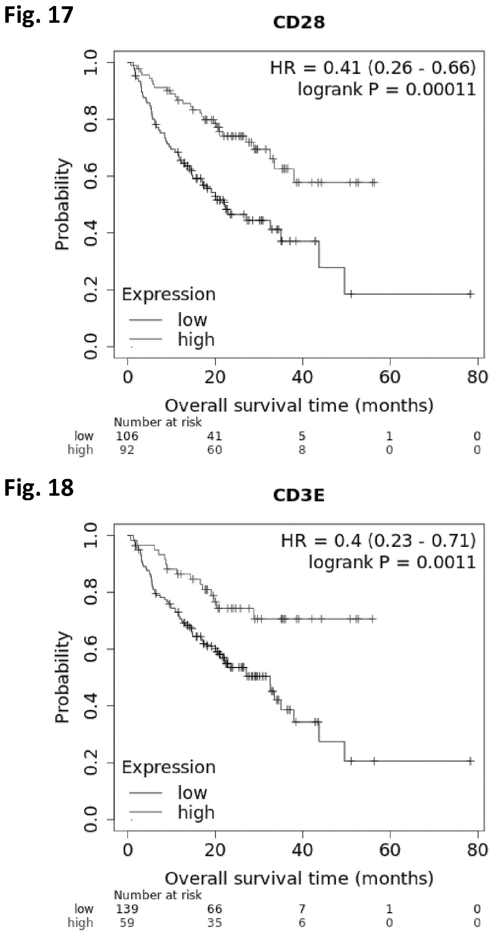

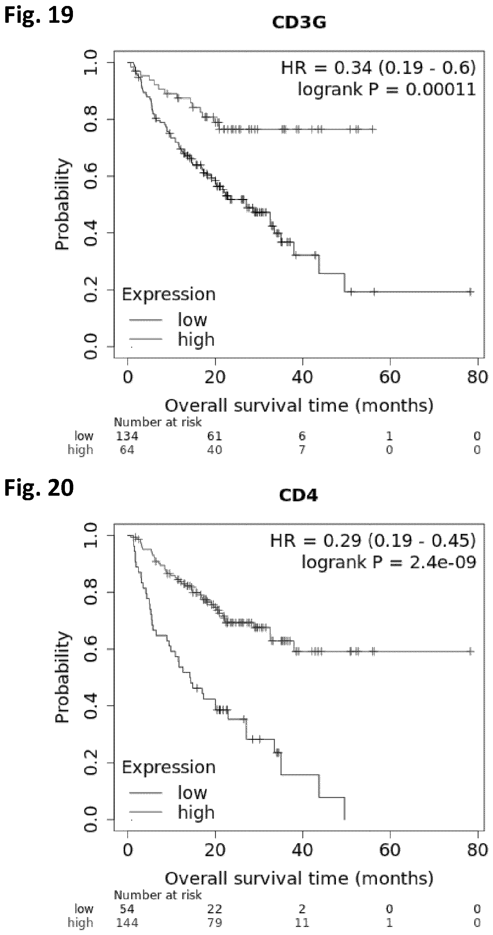

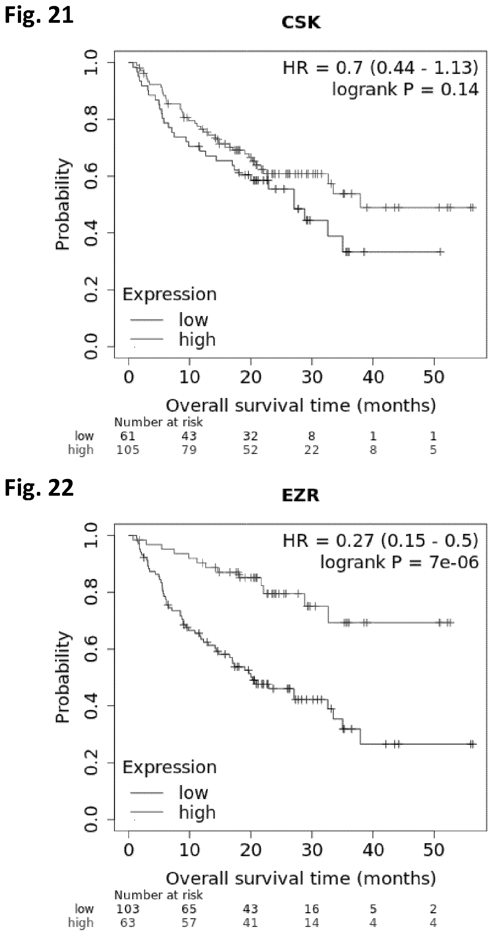

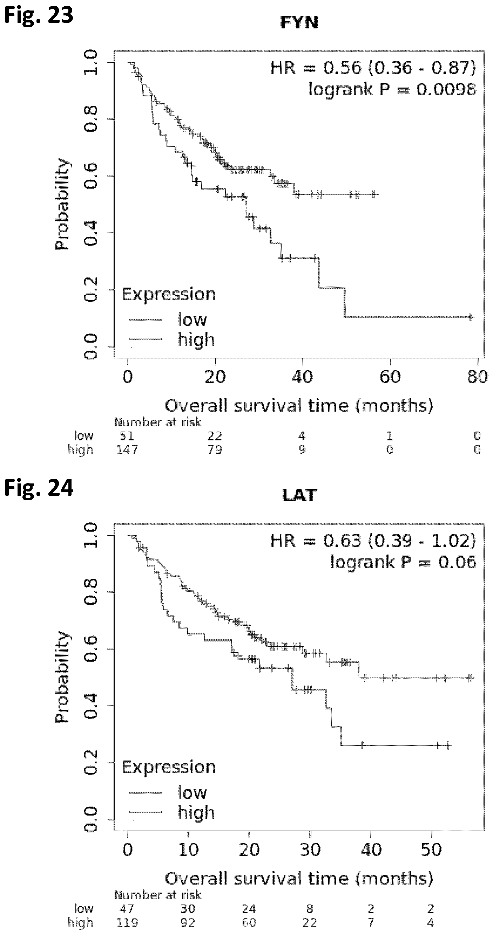

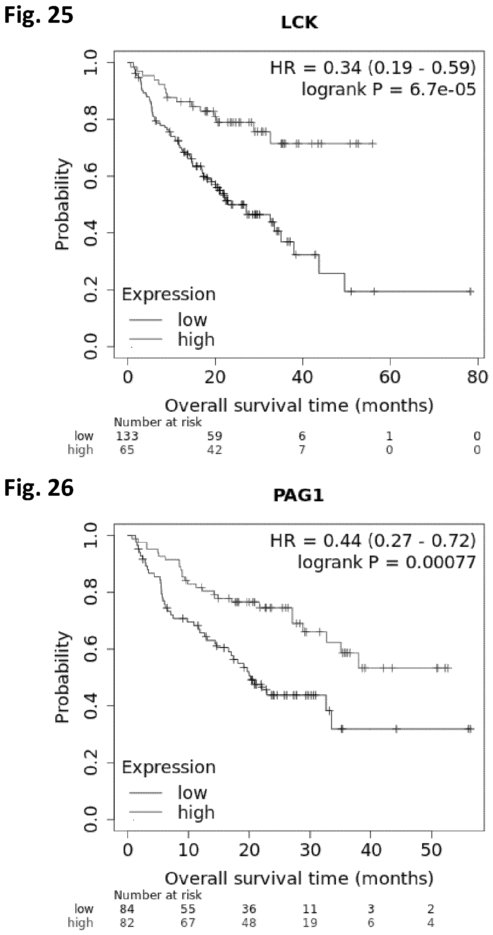

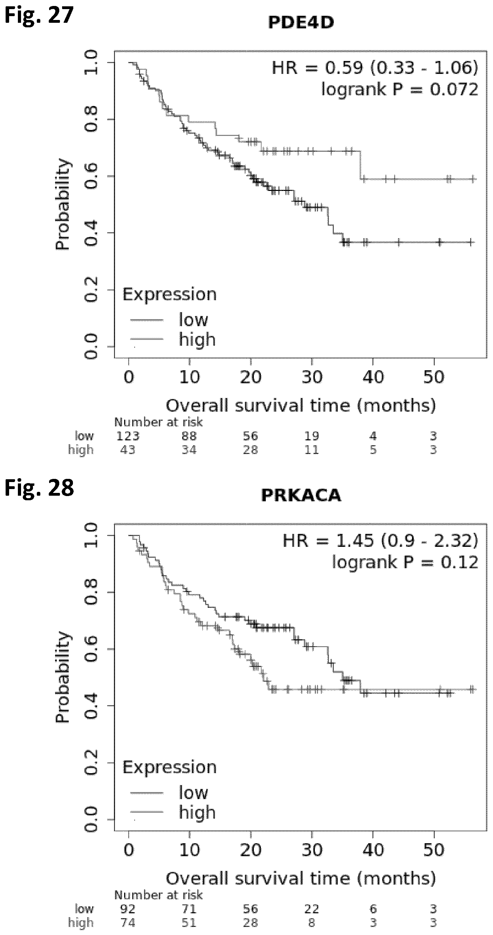

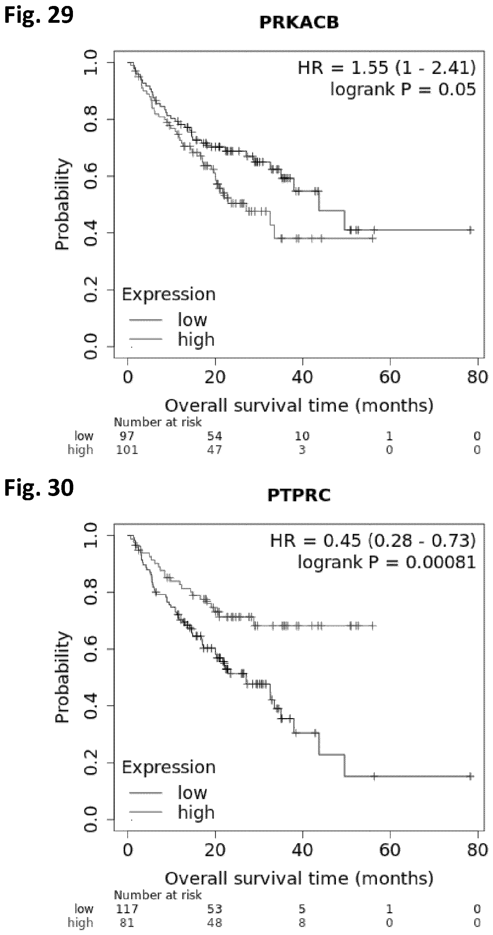

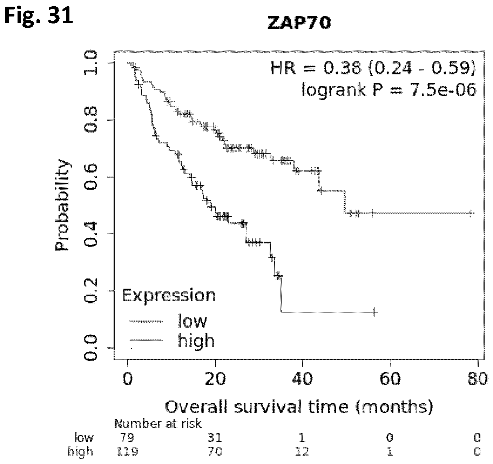

Figs. 1-33 show a Kaplan-Meier curve based on single gene expression (high or low) in a 198 patient cohort with all patients having melanoma. The clinical endpoint tested was overall survival time in months. Patients were stratified into 2 groups according to their risk of experiencing the clinical endpoint as predicted by the respective gene expression values. To select a cut-off for patient stratification into the two risk groups all possible cut-off values between the lower and the upper quartiles were computed and the best performing threshold was used. Logrank, HR and confidence interval are included in the figure. The included supplementary lists indicate the number of patients at risk. Figs. 1-31 respectively show prediction for the following genes (also indicated in the respective graphs): AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, and ZAP70.

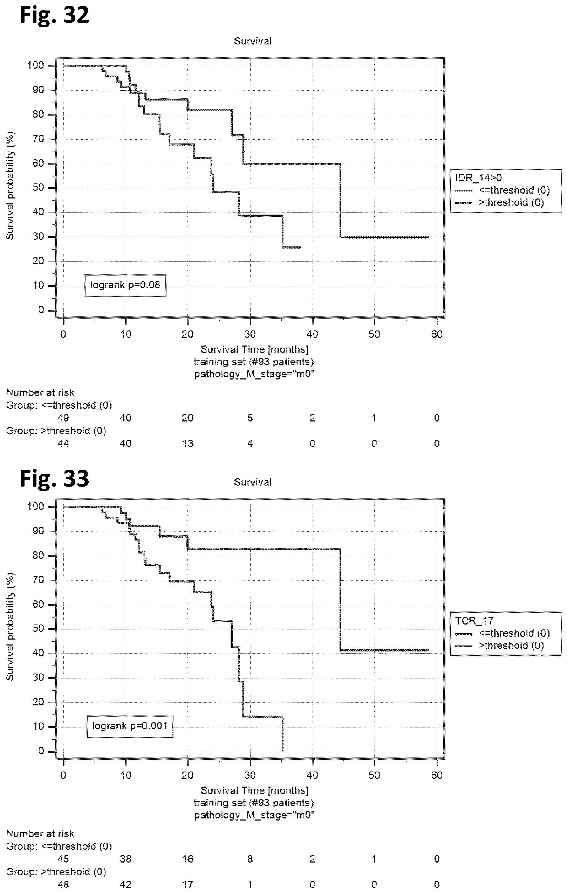

Fig. 32 shows a Kaplan-Meier curve of the IDR_14 model in a 93-patient training cohort with all patients having a melanoma. The clinical endpoint tested was overall survival time in months. Patients were stratified into 2 groups according to their risk of experiencing the clinical endpoint as predicted by the respective Cox regression model. The median value of the prognostic risk scores as derived from the Cox regression was used as a cut-off point for group stratification. Model parameters were established in a train set, and were fixed for validation in the test cohort (shown here). Logrank, HR and confidence interval are included in the figure. The included supplementary lists indicate the number of patients at risk.
Fig. 33 shows a Kaplan-Meier curve of the TCR_17 model in a 93-patient training cohort with all patients having a melanoma. The clinical endpoint tested was overall survival time in months. Patients were stratified into 2 groups according to their risk of experiencing the clinical endpoint as predicted by the respective Cox regression model. The median value of the prognostic risk scores as derived from the Cox regression was used as a cut-off point for group stratification. Model parameters were established in a train set, and were fixed for validation in the test cohort (shown here). Logrank, HR and confidence interval are included in the figure. The included supplementary lists indicate the number of patients at risk.

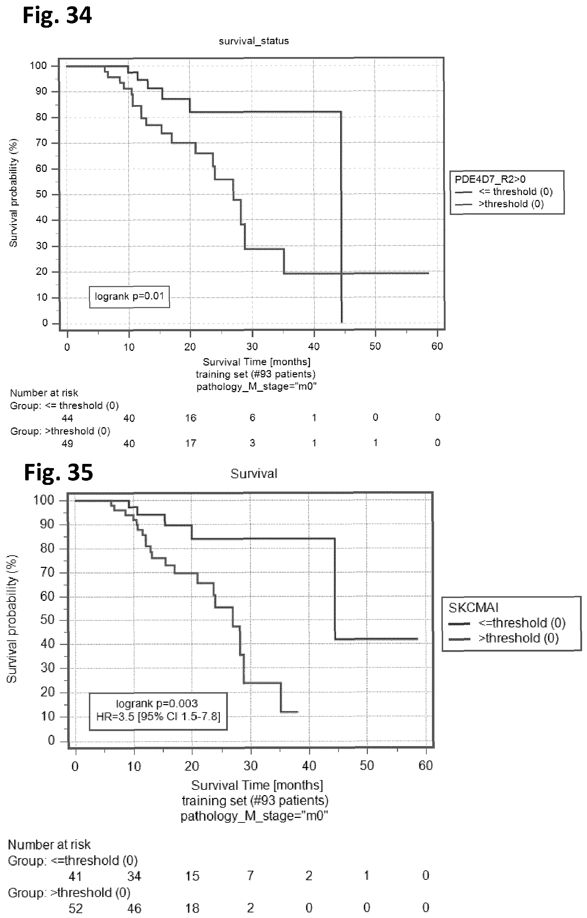

Fig. 34 shows a Kaplan-Meier curve of the PDE4D7 _R2 model in a 93-patient training cohort with all patients having a melanoma. The clinical endpoint tested was overall survival time in months. Patients were stratified into 2 groups according to their risk of experiencing the clinical endpoint as predicted by the respective Cox regression model. The median value of the prognostic risk scores as derived from the Cox regression was used as a cut-off point for group stratification. Model parameters were established in a train set, and were fixed for validation in the test cohort (shown here). Logrank, HR and confidence interval are included in the figure. The included supplementary lists indicate the number of patients at risk.
Fig. 35 shows a Kaplan-Meier curve of the SKCMAI model in a 93-patient training cohort with all patients having a melanoma. The clinical endpoint tested was overall survival time in months. Patients were stratified into 2 groups according to their risk of experiencing the clinical endpoint as predicted by the respective Cox regression model. The median value of the prognostic risk scores as derived from the Cox regression was used as a cut-off point for group stratification. Model parameters were established in a train set, and were fixed for validation in the test cohort (shown here). Logrank, HR and confidence interval are included in the figure. The included supplementary lists indicate the number of patients at risk.

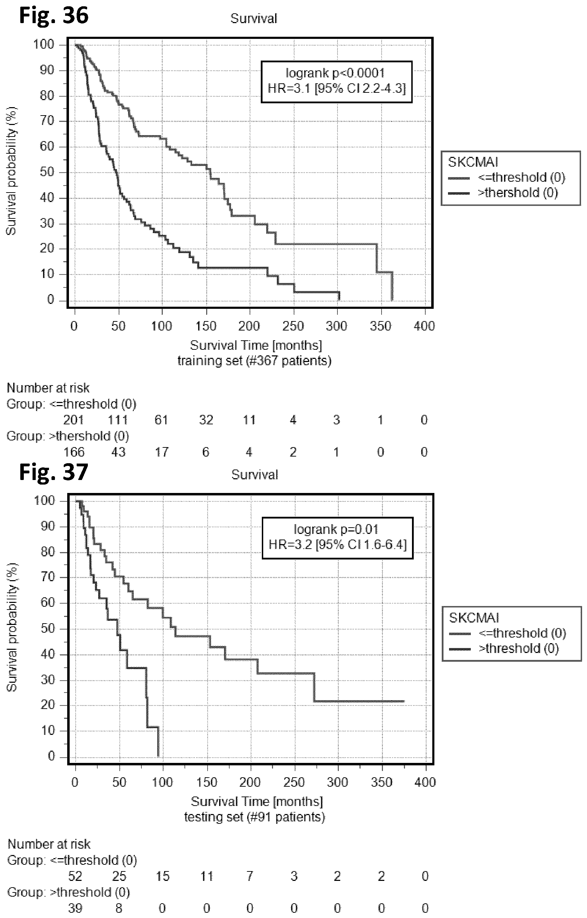

Figs. 36 and 37 show a Kaplan-Meier curves of the SKCMAI model in a 458-patient cohort with all patients having a melanoma. The cohort is split in a 367 patient training cohort (Fig. 36) and a 91 patient test cohort (Fig. 37). The clinical endpoint tested was overall survival time in months. Patients were stratified into 2 groups according to their risk of experiencing the clinical endpoint as predicted by the respective Cox regression model. The median value of the prognostic risk scores as derived from the Cox regression was used as a cut-off point for group stratification. Model parameters were established in a train set, and were fixed for validation in the test cohort (shown here). Logrank, HR and confidence interval are included in the figure. The included supplementary lists indicate the number of patients at risk.

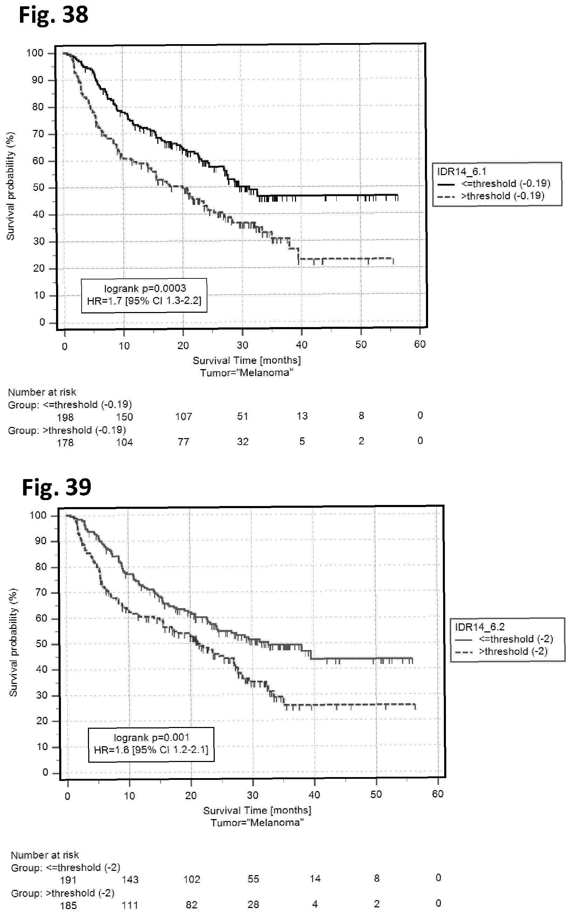

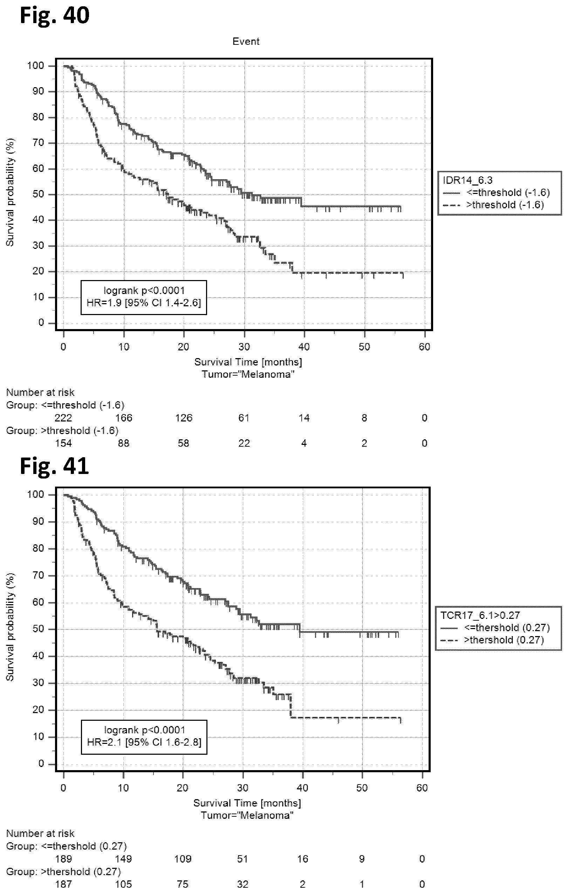

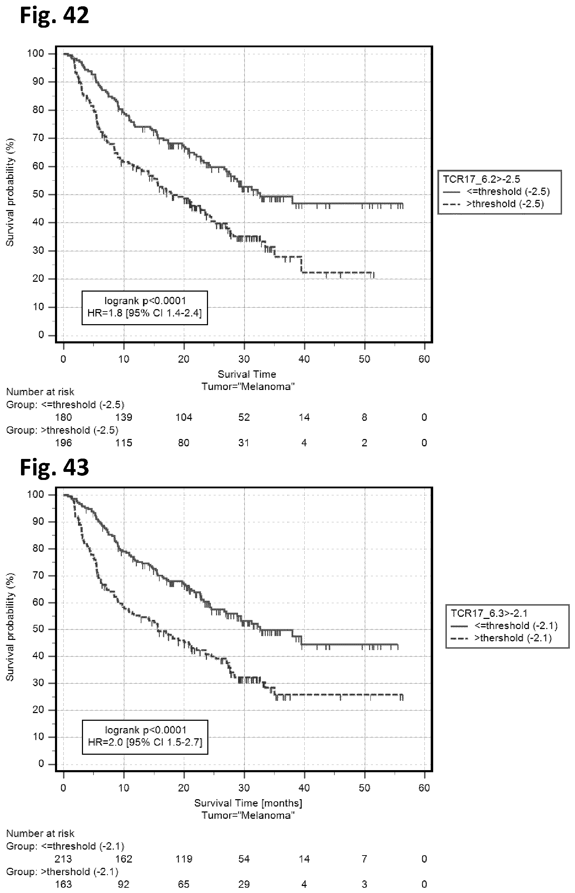

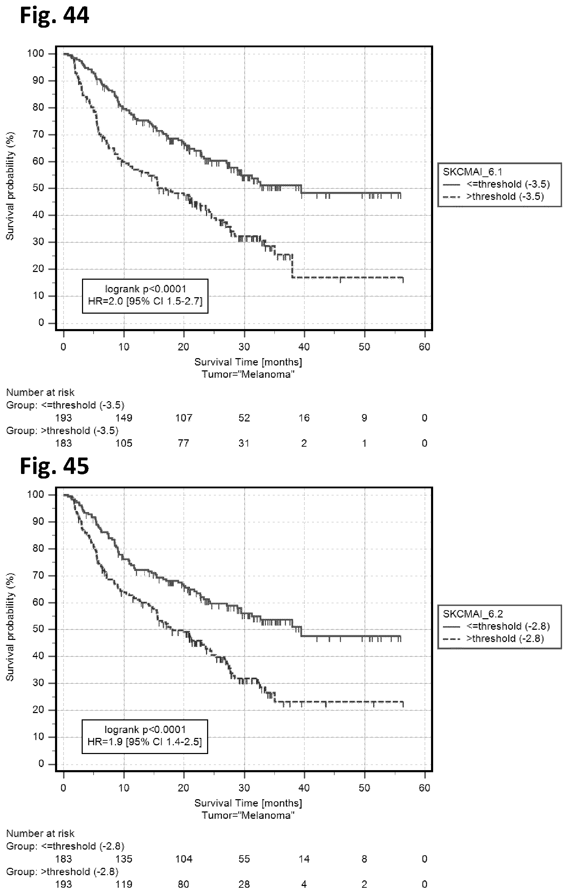

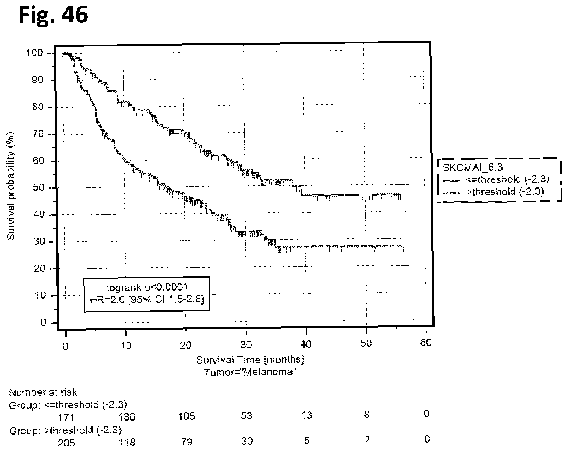

Figs. 38-46 show Kaplan-Meier curves of different 6-gene models in a 376-patient cohort with all patients having a melanoma and. The clinical endpoint tested was overall survival time in months. Patients were stratified into 2 groups according to their risk of experiencing the clinical endpoint as predicted by the respective Cox regression model. The median value of the prognostic risk scores as derived from the Cox regression was used as a cut-off point for group stratification. Model parameters were established in a train set, and were fixed for validation in the test cohort (shown here). Logrank, HR and confidence interval are included in the figure. The included supplementary lists indicate the number of patients at risk. The Figs. 38-46 respectively show the IDR_14 6.1, IDR_14 6.2, IDR_14 6.3, TCR_17 6.1, TCR_17 6.2, TCR_17 6.3, SKCMAI 6.1, SKCMAI 6.2, and SKCMAI 6.1 models as indicated and described herein.

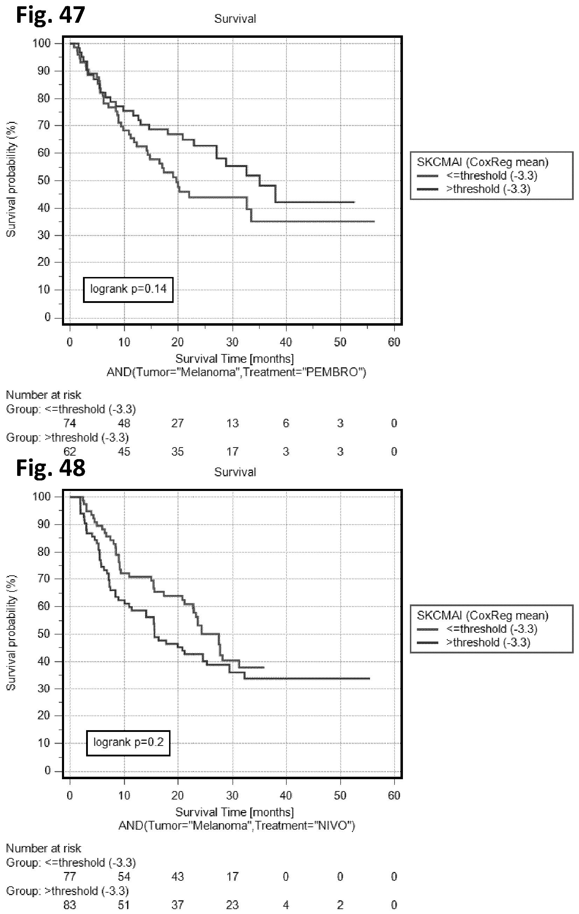

Figs. 47 and 48 show Kaplan-Meier curves of the SKCMAI model in a 296-patient cohort with all patients having a melanoma and being treated with immune checkpoint inhibitors. The cohort is split into patients being treated with Pembrolizumab (Fig. 47, 136 patients) or Nivolumab (Fig. 48, 160 patients). The clinical endpoint tested was overall survival time in months. Patients were stratified into 2 groups according to their risk of experiencing the clinical endpoint as predicted by the respective Cox regression model. The median value of the prognostic risk scores as derived from the Cox regression was used as a cut-off point for group stratification. Model parameters were established in a train set, and were fixed for validation in the test cohort (shown here). Logrank, HR and confidence interval are included in the figure. The included supplementary lists indicate the number of patients at risk.

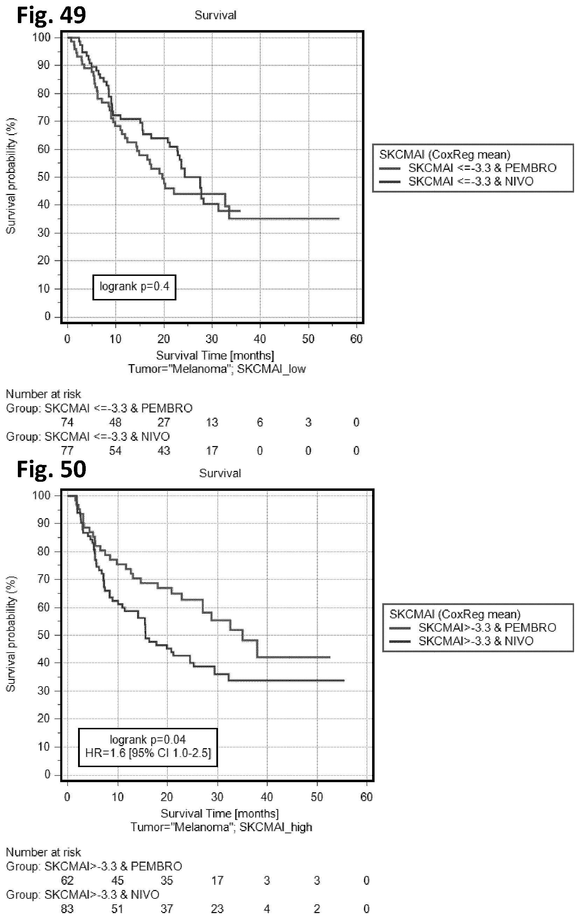

Figs. 49 and 50 show Kaplan-Meier curves of the SKCMAI model in a 296-patient cohort with all patients having a melanoma and being treated with immune checkpoint inhibitors. The cohort is split into patients groups according to their SKCMAI risk profile being favorable (Fig. 49, low SKCMAI score is favorable outcome; 151 patients) or unfavorable (Fig. 50, high SKCMAI score is unfavorable outcome; 145 patients). The clinical endpoint tested was overall survival time in months. Patients were stratified into 2 groups according to the received immune checkpoint inhibitor, Pembrolizumab or Nivolumab. The median value of the prognostic risk scores as derived from the Cox regression was used as a cut-off point for group stratification. Model parameters were established in a train set, and were fixed for validation in the test cohort (shown here). Logrank, HR and confidence interval are included in the figure. The included supplementary lists indicate the number of patients at risk.

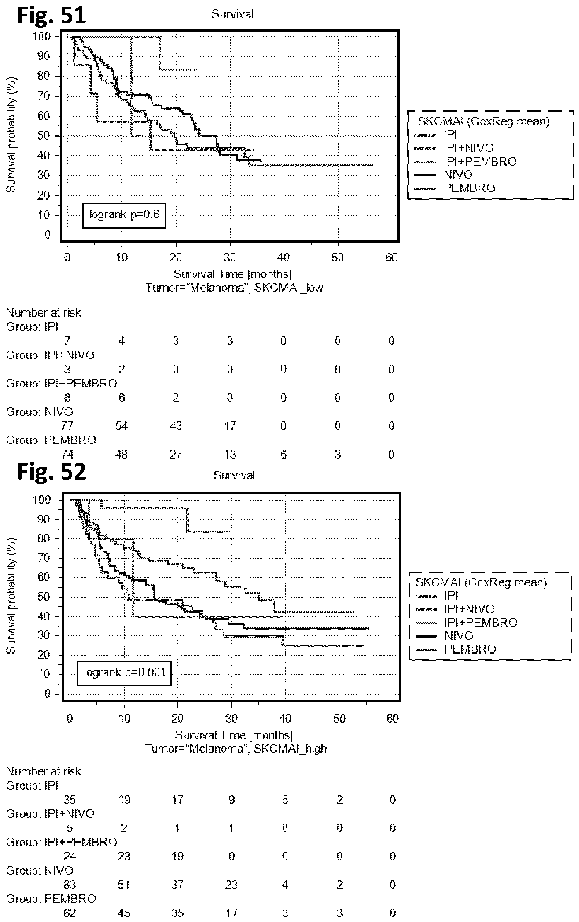

Figs. 51 and 52 show Kaplan-Meier curves of the SKCMAI model in a 376-patient cohort with all patients having a melanoma and being treated with immune checkpoint inhibitors. The cohort is split into patients groups according to their SKCMAI risk profile being favorable (Fig. 50, low SKCMAI score is favorable outcome; 167 patients) or unfavorable (Fig. 52, high SKCMAI score is unfavorable outcome; 209 patients). The clinical endpoint tested was overall survival time in months. Patients were stratified into 5 groups according to the received treatment, Ipilimumab only or Pembrolizumab or Nivolumab either with or without Ipilimumab. The median value of the prognostic risk scores as derived from the Cox regression was used as a cut-off point for group stratification. Model parameters were established in a train set, and were fixed for validation in the test cohort (shown here). Logrank, HR and confidence interval are included in the figure. The included supplementary lists indicate the number of patients at risk.

DEFINITIONS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of $\pm 20\%$, preferably $\pm 15\%$, more preferably $\pm 10\%$, and even more preferably $\pm 5\%$.

**"About" and "approximately":** these terms, when referring to a measurable value such as an amount, a temporal duration, and the like, is meant to encompass variations of ±20% or ± 10%, more preferably ±5%, even more preferably ±1%, and still more preferably ±0.1% from the specified value, as such variations are appropriate to perform the disclosed methods.

**"Antagonist"** and **"inhibitor":** These terms are used interchangeably, and they refer to a compound or agent having the ability to reduce or inhibit a biological function of a target protein or polypeptide, such as by reducing or inhibiting the activity or expression of the target protein or polypeptide. Accordingly, the terms "antagonist" and "inhibitor" are defined in the context of the biological role of the target protein or polypeptide. An inhibitor need not completely abrogate the biological function of a target protein or polypeptide, and in some embodiments reduces the activity by at least 20%, 30%, 40%, 50%, 60%, 70%, 80%, 90%, 95%, or 99%. While some antagonists herein specifically interact with (e.g., bind to) the target, compounds that inhibit a biological activity of the target protein or polypeptide by interacting with other members of the signal transduction pathway of which the target protein or polypeptide are also specifically included within this definition. Non-limiting examples of biological activity inhibited by an antagonist include those associated with the development, growth, or spread of a tumor, or an undesired immune response as manifested in autoimmune disease.

**"Anti-cancer effect":** This refers to the effect a therapeutic agent has on cancer, e.g., a decrease in growth, viability, or both of a cancer cell. The IC50 of cancer cells can be used as a measure the anti-cancer effect. IC50 refers to a measure of the effectiveness of a therapeutic agent in inhibiting cancer cells by 50%.

**"Alleviating cancer":** The term, in the context of specific cancers and/or their pathologies, refers to degrading a tumor, for example, breaking down the structural integrity or connective tissue of a tumor, such that the tumor size is reduced when compared to the tumor size before treatment. "Alleviating" metastasis of cancer includes reducing the rate at which the cancer spreads to other organs.

**"Compositions", "products" or "combinations":** These encompass those compositions suitable for various routes of administration, including, but not limited to, intravenous, subcutaneous, intradermal, subdermal, intranodal, intratumoral, intramuscular, intraperitoneal, oral, nasal, topical (including buccal and sublingual), rectal, vaginal, aerosol and/or parenteral or mucosal application. The compositions, formulations, and products according to the disclosure invention normally comprise the drugs/compound/inhibitor (alone or in combination) and one or more suitable pharmaceutically acceptable excipients or carriers.

**"Combination therapy", or "in combination with":** These term refers to the use of more than one compound or agent to treat a particular disorder or condition. For example, Compound 1 may be administered in combination with at least one additional therapeutic agent. By "in combination with," it is not intended to imply that the other therapy and Compound 1 must be administered at the same time and/or formulated for delivery together, although these methods of delivery are within the scope of this disclosure. Compound 1 can be administered concurrently with, prior to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks before), or subsequent to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks after), one or more other additional agents. In general, each therapeutic agent will be administered at a dose and/or on a time schedule determined for that particular agent. The other therapeutic agent can be administered with Compound 1 herein in a single composition or separately in a different composition. Higher combinations, e.g., triple therapy, are also contemplated herein.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, the term "and/or" indicates that one or more of the stated cases may occur, alone or in combination with at least one of the stated cases, up to with all of the stated cases.

As used herein, the term "conventional techniques" refers to a situation wherein the methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

As used herein, the term "identity" refers to a measure of the identity of nucleotide sequences or amino acid sequences. In general, the sequences are aligned so that the highest order match is obtained. "Identity" per se has an art-recognized meaning and can be calculated using published techniques. See, e.g.: (Computational Molecular Biology, Lesk, A. M., ED., Oxford University Press, New York, 1988; Biocomputing: Informatics And Genome Projects, Smith, D. W., ED., Academic Press, New York, 1993; Computer Analysis Of Sequence Data, Part I, Griffin, A. M., And Griffin, H. G., EDS., Humana Press, New Jersey, 1994; Sequence Analysis In Molecular Biology, Von Heinje, G., Academic Press, 1987; and Sequence Analysis Primer; Gribskov, M. and Devereux, J., eds., M Stockton Press, New York, 1991). While there exist a number of methods to measure identity between two nucleotide sequences or amino acid sequences, the term "identity" is well known to skilled artisans (Carillo, H., and Lipton, D., SIAM J. Applied Math (1988) 48:1073). Methods commonly employed to determine identity or similarity between two sequences include, but are not limited to, those disclosed in Guide To Huge Computers, Martin J. Bishop, ed., Academic Press, San Diego, 1994, and Carillo, H., and Lipton, D., Siam J. Applied Math (1988) 48:1073. Methods to determine identity and similarity are codified in computer programs. Preferred computer program methods to determine identity and similarity between two sequences include, but are not limited to, GCG program package (Devereux, J., et al., Nucleic Acids Research (1984) 12(1):387), BLASTP, BLASTN, FASTA (Atschul, S. F. et al., J. Molec. Biol. (1990) 215:403).

As an illustration, by a polynucleotide having a nucleotide sequence having at least, for example, 95% "identity" to a reference nucleotide sequence encoding a polypeptide of a certain sequence, it is intended that the nucleotide sequence of the polynucleotide is identical to the reference sequence except that the polynucleotide sequence may include up to five point mutations per each 100 nucleotides of the reference amino acid sequence. In other words, to obtain a polynucleotide having a nucleotide sequence at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. These mutations of the reference sequence may occur at the 5' or 3' terminal positions of the reference nucleotide sequence, or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO: X is intended that the amino acid sequence of the polypeptide is identical to the reference sequence except that the amino acid sequence may include up to five amino acid alterations per each 100 amino acids of the reference amino acid of SEQ ID NO: X. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

The terms sequency identity, sequence homology, and x percent identical to are used herein interchangeably and intend to refer to the same as defined above.

As used herein, the term "in vitro" refers to experimentation or measurements conducted using components of an organism that have been isolated from their natural conditions.

As used herein, the term "ex vivo" refers to experimentation or measurements done in or on tissue from an organism in an external environment with minimal alteration of natural condition.

As used herein, the term "nucleic acid", "nucleic acid molecule" and "polynucleotide" is intended to include DNA molecules and RNA molecules. A nucleic acid (molecule) may be single-stranded or double-stranded, but preferably is double-stranded DNA.

As used herein, the terms "sequence" when referring to nucleotides, or "nucleic acid sequence", "nucleotide sequence" or "polynucleotide sequence" refer to the order of nucleotides of, or within, a nucleic acid and/or polynucleotide. Within the context of the current invention a first nucleic acid sequence may be comprised within or overlap with a further nucleic acid sequence.

As used herein, the term "subject" or "individual" or "animal" or "patient" or "mammal," used interchangeably, refer to any subject, particularly a mammalian subject, for whom diagnosis, prognosis, or therapy is desired. Mammalian subjects include humans, domestic animals, farm animals, and zoo-, sports-, or pet-animals such as dogs, cats, guinea pigs, rabbits, rats, mice, horses, cattle, cows, bears, and so on. As defined herein a subject may be alive or dead. Samples can be taken from a subject post-mortem, i.e. after death, and/or samples can be taken from a living subject.

As used herein, terms "treatment", "treating", "palliating", "alleviating" or "ameliorating", used interchangeably, refer to an approach for obtaining beneficial or desired results including, but not limited to, therapeutic benefit. By therapeutic benefit is meant eradication or amelioration or reduction (or delay) of progress of the underlying disease being treated. Also, a therapeutic benefit is achieved with the eradication or amelioration or reduction (or delay) of progress of one or more of the physiological symptoms associated with the underlying disease such that an improvement or slowing down or reduction of decline is observed in the patient, notwithstanding that the patient can still be afflicted with the underlying disease.

Herein, the term "outcome" relates to a specific result or effect that can be measured. Examples of an outcome of a subject having melanoma include an outcome of the melanoma, a pathology outcome, an outcome of a therapy for treating melanoma, such as a surgery outcome, a radiation therapy outcome, a chemotherapy outcome, and an immunotherapy outcome, as well as other outcomes, such as a biomarker related, a genomic profile related outcome, an imaging related outcome (e.g., a change in morphology of the tumor), a biology related outcome (e.g., inflammation or immune response), a surrogate marker related outcome, a treatment side effect outcome, a treatment toxicity outcome, a disease pain outcome, a quality of life outcome, a cancer specific survival, and an overall survival.

The term "clinical recurrence" refers to the presence of clinical signs indicating the presence of tumour cells as measured, for example using in vivo imaging.

When used herein, the term "immune defense response genes" is interchangeably used with "IDR genes", "IDR_14" or "immune defense genes" and refers to one or more of the genes selected from AIM2, APOBEC3A, CIAO1, DDX58, DHX9, IFI16, IFIH1, IFIT1, IFIT3, LRRFIP1, MYD88, OAS1, TLR8, and ZBP1.

When used herein, the term "T-cell receptor signalling genes" is interchangeably used with "TCR signalling genes", "TCR_17" or "TCR genes" and refers to one or more of the genes selected from: CD2, CD247, CD28, CD3E, CD3G, CD4, CSK, EZR, FYN, LAT, LCK, PAG1, PDE4D, PRKACA, PRKACB, PTPRC, and ZAP70.

When used herein, the term "SKCMAI genes" is interchangeably used with "Skin cancer melanoma AI model genes", "SKCMAI" or "SKCMAI model" and refers to one or more of the genes selected from: AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, and ZAP70.

DETAILED DESCRIPTION OF EMBODIMENTS

The section headings as used herein are for organizational purposes only and are not to be construed as limiting the subject matter described.

A portion of this invention contains material that is subject to copyright protection (such as, but not limited to, diagrams, device photographs, or any other aspects of this submission for which copyright protection is or may be available in any jurisdiction). The copyright owner has no objection to the facsimile reproduction by anyone of the patent document or patent invention, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention relates, unless otherwise indicated. Other specifically defined terms are to be construed in a manner consistent with the definition as provided herein. The preferred materials and methods are described herein, although any methods and materials similar or equivalent to those described herein can be used in the practice for testing of the present invention. Unless otherwise defined, all technical and scientific terms used herein have the same meaning as commonly understood by a person of ordinary skill in the art.

The invention broadly relates to the realization that previously identified gene signatures may be used to predict an outcome for patients suffering from a melanoma. The gene signatures can further be used to predict a response to treatment with an immune checkpoint inhibitor (ICI) for patients suffering from a melanoma. The gene signatures have initially been described as relevant for prostate cancer (WO2022043299A1, WO2021175986A1, both hereby incorporated by reference in its entirety), but as shown herein, have also surprisingly a predictive effect for treatment outcome with an ICI for subjects suffering from melanoma, or to predict a general outcome for melanoma patients. To demonstrate this the inventors have analyzed RNA sequencing data from melanoma patients and particularly melanoma patients treated with ICI and correlated gene expression data of the gene signatures with the available follow up data, such as survival. The inventors show that almost all of the Immune response defense gene signature (IDR14) and the T Cell receptor signaling Signature (TCR17) are individually predictive for an outcome, as demonstrated in Figs. 1 to 31. In addition the prediction could be further improved by combining several genes from the IDR14 and TCR17 gene signatures, see Figs. 32-37 (IDR_14 model, TCR_17 model, SKCMAI model) and Figs. 38-46 (6-gene models). In the experiments represented by these figures, random selections of 6 genes were made from the IDR14 gene signature, the TCR17 gene signature, or the combined pool of genes of both the IDR14 and TCR17 gene signatures (herein referred to as SKCMAI). This makes it plausible that each selection of six genes of the gene signatures individually or the combined gene signatures is predictive for an outcome of treatment with an ICI of a subject having melanoma. Thus, using one or six gene models or a full SKCMAI model melanoma patients undergoing ICI treatment can be stratified in a subgroup having a poor predicted outcome (high SKCMAI score) and a subgroup having a good predicted outcome (low SKCMAI score).

Lastly the inventors further reviewed response to individual ICI treatments, particularly Pembroluzimab and Nivolumab. For the patients having a low SKCMAI score (in general corresponding to a favorable predicted outcome), no significant outcome difference in response to Pembroluzimab vs. Nivolumab were found while in patients having a high SKCMAI score (in general corresponding to an unfavorable predicted outcome), a significant better outcome is observed when the patients were treated with Pembrolizumab instead of Nivolumab (see Figs. 47-52). These results suggest that the genes and signatures described herein can be used to provide treatment suggestions in patients having a high SKCMAI corresponding to an unfavorable predicted outcome as these patients will likely benefit from treatment with Pembrolizumab while treatment success may fail if these patients are treated with Nivolumab.

Therefore, in a first aspect the invention relates to Pembroluzimab or an equivalent thereof for use in the treatment of a melanoma in a subject, the use comprising: determining or receiving the result of a determination of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and determining the prediction of the outcome based on one or more gene expression levels, wherein the outcome is a poor outcome or a good outcome, and when the predicted outcome is a poor outcome Pembroluzimab or an equivalent thereof is administered to the subject.

Alternatively the invention relates to a method for treating a subject suffering from melanoma, the method comprising determining or receiving the result of a determination of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and determining the prediction of the outcome based on one or more gene expression levels, wherein the outcome is a poor outcome or a good outcome, and when the predicted outcome is a poor outcome Pembroluzimab or an equivalent thereof is administered to the subject.

Melanoma is a type of cancer which is derived from melanocytes. Typically melanoma starts in the skin and is also called cutaneous melanoma, however melanomas may also start in other parts of the body such as inside the eye (ocular melanomas) or inside the nose, mouth, throat, genital or anal area (mucosal melanomas). Different types of cutaneous melanoma are recognized, the most common ones being Superficial spreading melanoma, Nodular melanoma, Lentigo maligna melanoma and Acral lentiginous melanoma (acral melanoma).

*Superficial spreading melanoma*: This type makes up about 7 in 10 melanomas of the skin. These tumors tend to grow outward on the surface of the skin (at least at first), and might be noticed as a dark spot on the skin that is changing shape and/or getting bigger. Some of these melanomas start in existing moles.

*Nodular melanoma*: This type accounts for about 2 in 10 skin melanomas. These tumors often appear as a distinct, raised bump (nodule) on the skin that is often dark brown or black, but it can also be pink or red. Nodular melanomas tend to grow down into deeper layers of the skin fairly early, so they're often at a more advanced stage than superficial spreading melanomas by the time they are found.

*Lentigo maligna melanoma*: This type of melanoma tends to occur in older people. It often first appears as an abnormally shaped tan or brown spot in an area that gets a lot of sun, and it tends to grow slowly over time.

Acral lentiginous melanoma (acral melanoma): This type of melanoma starts in areas that don't get a lot of sun exposure, such as the palms of the hands, soles of the feet, or under the nails.

Risk factors for developing melanoma include UV light exposure, moles, light skin, hair or eye color, genetics, weakened immune system, age and gender (with older and male subjects being more predisposed).

The most frequent mutation occurs in the 600th codon of BRAF (50% of cases). BRAF is normally involved in cell growth, and this specific mutation renders the protein constitutively active and independent of normal physiological regulation, thus fostering tumor growth. RAS genes (NRAS, HRAS and KRAS) are also recurrently mutated (30% of TCGA cases) and mutations in the 61st or 12th codons trigger oncogenic activity. Loss-of-function mutations often affect tumor suppressor genes such as NF1, TP53 and CDKN2A. Other oncogenic alterations include fusions involving various kinases such as BRAF, RAF1, ALK, RET, ROS1, NTRK1, NTRK3 and MET.

Melanomas can be subdivided in stages depending on the progress of the disease:

Melanoma in situ is also called stage 0 melanoma meaning the cancer cells are contained in the top layer of skin.
Stage 1 melanoma is at an early stage. It's only in the skin and there's no sign that it has spread to lymph nodes or other parts of the body.
Stage 2 melanoma is only in the skin and there is no sign that it has spread to lymph nodes or other parts of the body.

Stage 3 generally means melanoma has spread to nearby lymph nodes. Or it has spread to an area between the melanoma and the nearby lymph nodes.
Stage 4 melanoma has spread elsewhere in the body, away from the primary site and the nearby lymph nodes.

Treatment of melanoma depends on the stage. Stage 0 melanoma (melanoma in situ) has not grown deeper than the top layer of the skin (the epidermis). It is usually treated by surgery (wide excision) to remove the melanoma and a small margin of normal skin around it. The removed sample is then sent to a lab to be looked at with a microscope. If cancer cells are seen at the edges of the sample, a second, wider excision of the area may be done. The use of imiquimod cream (Zyclara) or radiation therapy after surgery may be considered if not all the cancer cells can be removed for some reason.

Stage I melanomas have grown into deeper layers of the skin, but they haven't grown beyond the area where they started. These cancers are typically treated by wide excision (surgery to remove the tumor as well as a margin of normal skin around it). The width of the margin depends on the thickness and location of the melanoma. Most often, no other treatment is needed. In some cases a sentinel lymph node biopsy (SLNB) may be performed to look for cancer in nearby lymph nodes, especially if the melanoma is stage IB or has other traits that make it more likely to have spread. If the SLNB does not find cancer cells in the lymph nodes, then no further treatment is needed, although close follow-up is still important. If cancer cells are found on the SLNB (which changes the cancer stage to stage III - see below), a lymph node dissection (removal of all lymph nodes near the cancer) might be recommended. Another option might be to watch the lymph nodes closely by getting an imaging test such as ultrasound of the nodes every few months. If the SLNB found cancer, adjuvant (additional) treatment with immune checkpoint inhibitors or targeted therapy drugs (if the melanoma has a BRAF gene mutation) might be recommended to try to lower the chance the melanoma will come back.

Stage II melanomas have grown deeper into the skin than stage I melanomas, but they still haven't grown beyond the area in the skin where they started. Wide excision (surgery to remove the melanoma and a margin of normal skin around it) is the standard treatment for these cancers. The width of the margin depends on the thickness and location of the melanoma. Because the melanoma may have spread to nearby lymph nodes, a sentinel lymph node biopsy (SLNB) is recommended as well. If a SLNB is done and does not find cancer cells in the lymph nodes, then sometimes no further treatment is needed, but close follow-up is still important. For certain stage II melanomas, the immune checkpoint inhibitor pembrolizumab (Keytruda) might be given after surgery to help reduce the risk of the cancer returning. Radiation therapy to the area might be another option, especially if the melanoma has features that make it more likely to come back. If the SLNB finds that the sentinel node contains cancer cells (which changes the cancer stage to stage III - see below), then a lymph node dissection (where all the lymph nodes in that area are surgically removed) might be recommended. Another option might be to watch the lymph nodes closely with an imaging test such as ultrasound of the nodes every few months. Whether or not the lymph nodes are removed, adjuvant (additional) treatment with immune checkpoint inhibitors or targeted therapy drugs (if the melanoma has a BRAF gene mutation) might be recommended to try to lower the chance the melanoma will come back.

Stage III melanomas have spread to nearby areas in the skin or lymph vessels, or they have reached the nearby lymph nodes. Surgical treatment for stage III melanoma usually requires wide excision of the primary tumor as in earlier stages, along with a lymph node dissection (where all the nearby lymph nodes are surgically removed). After surgery, (additional) adjuvant treatment with immune checkpoint inhibitors or with targeted therapy drugs (for cancers with BRAF gene changes) may help lower the risk of the melanoma coming back. Other drugs or perhaps vaccines may also be recommended as part of a clinical trial to try to reduce the chance the melanoma will come back. Another option is to give radiation therapy to the areas where the lymph nodes were removed, especially if many of the nodes contain cancer. If melanoma tumors are found in nearby lymph vessels in or just under the skin (known as in-transit tumors), they are removed, if possible. Other options might include injections of the T-VEC vaccine (Imlygic), interleukin-2 (IL-2), or Bacille Calmette-Guerin (BCG) vaccine directly into the melanoma; radiation therapy; or applying imiquimod cream. For melanomas on an arm or leg, another option might be isolated limb perfusion or isolated limb infusion (infusing just the limb with chemotherapy). Other possible treatments might include targeted therapy drugs (for melanomas with a BRAF or C-KIT gene change), immunotherapy, or chemotherapy.

Stage IV melanomas have already spread (metastasized) to other parts of the body, such as distant lymph nodes, areas of skin, or other organs. Skin tumors or enlarged lymph nodes causing symptoms can often be removed by surgery or treated with radiation therapy. If there are only a few metastases, surgery to remove them might sometimes be an option, depending on where they are and how likely they are to cause symptoms. Metastases that can't be removed may be treated with radiation or with injections of the T-VEC vaccine (Imlygic) directly into the tumors. In either case, this is often followed by adjuvant treatment with medicines such as immunotherapy or targeted therapy drugs. The treatment of widespread melanomas has changed in recent years as newer forms of immunotherapy and targeted drugs have been shown to be more effective than chemotherapy.

In about half of all melanomas, the cancer cells have BRAF gene changes. These melanomas often respond to treatment with targeted therapy drugs - typically a combination of a BRAF inhibitor and a MEK inhibitor. However, the immune checkpoint inhibitors mentioned above are often tried first, as this seems to be more likely to help for longer periods of time. Another option might be a combination of targeted drugs plus the immune checkpoint inhibitor atezolizumab (Tecentriq). While immunotherapy is often used before targeted therapy, there might be situations where it makes sense to use targeted therapy first. For example, the targeted drugs are more likely to shrink tumors quickly, so they might be preferred in cases where this is important. In either case, if one type of treatment isn't working, the other can be tried. A small portion of melanomas have changes in the C-KIT gene. These melanomas might be helped by targeted drugs such as imatinib (Gleevec) and nilorinib (Tasigna), although these drugs often stop working eventually. Rarely, melanomas might have changes in other genes such as NRAS, ROS1, ALK, or the NTRK genes, which can be treated with targeted drugs.

Immunotherapy using other medicines might be an option if immune checkpoint inhibitors or other treatments aren't working. Options might include: Interleukin-2 (IL-2) (also known as aldesleukin) and Lifileucel (Amtagvi), a type of tumor-infiltrating lymphocyte (TIL) therapy.

Chemotherapy (chemo) can help some people with stage IV melanoma, but other treatments are usually tried first. Dacarbazine (DTIC) and temozolomide (Temodar) are the chemo drugs used most often, either by themselves or combined with other drugs. Even when chemo shrinks these cancers, the cancer usually starts growing again over time.

Treatment of melanoma that comes back after initial treatment depends on where in the body the melanoma is, what treatments a person has already had, the person's overall health and preferences, and other factors.

Local recurrence - Melanoma might come back in the skin near the site of the original tumor, sometimes even in the scar from the surgery. In general, these local (skin) recurrences are treated with surgery similar to what would be recommended for a primary melanoma. This might include a sentinel lymph node biopsy (SLNB). Depending on the results of the SLNB, other treatments might be recommended as well.

In-transit recurrence - If melanoma recurs in nearby lymph vessels in or just under the skin (known as in-transit recurrence), it should be removed with surgery, if possible. Other options might include injections of the T-VEC vaccine (Imlygic), interleukin-2 (IL-2), or Bacille Calmette-Guerin (BCG) vaccine directly into the melanoma; radiation therapy; or applying imiquimod cream. For melanomas on an arm or leg, another option might be isolated limb perfusion or isolated limb infusion (infusing just the limb with chemotherapy). Other treatments might include targeted therapy (for melanomas with a BRAF or C-KIT gene change), immunotherapy, or chemotherapy.

Recurrence in nearby lymph nodes - If the nearby lymph nodes weren't removed during the initial treatment, the melanoma might come back in these lymph nodes. Lymph node recurrence is typically treated by lymph node dissection if it can be done, sometimes followed by adjuvant (additional) treatments such as radiation therapy and/or immunotherapy or targeted therapy (for cancers with BRAF gene changes). If surgery is not an option, radiation therapy or systemic treatment (immunotherapy, targeted therapy, or chemo) can be used.

Recurrence in other parts of the body - Melanoma might also come back in distant parts of the body. Almost any organ can be affected. Most often, the melanoma comes back in the lungs, bones, liver, or brain. Treatment for these recurrences is generally the same as for stage IV melanoma (see above). Melanomas that recur on an arm or leg may be treated with isolated limb perfusion/infusion chemotherapy.

The following immune checkpoint inhibitors are typically used in treatment of melanoma:
PD-1 inhibitors - Pembrolizumab (Keytruda) and nivolumab (Opdivo) are drugs that target PD-1, a protein on immune system cells called T cells that normally helps keep these cells from attacking other cells in the body. By blocking PD-1, these drugs boost the immune response against melanoma cells. They can be used to treat melanomas that cannot be removed by surgery or that have spread to other parts of the body. They can further be used after surgery (as adjuvant treatment) for certain stage II, III, or IV melanomas that have been removed completely, to try to lower the risk of the cancer coming back. These drugs are given as an intravenous (IV) infusion, typically every 2 to 6 weeks, depending on the drug and why it's being given.

PD-L1 inhibitor - Atezolizumab (Tecentriq) is a drug that targets PD-L1, a protein related to PD-1 that is found on some tumor cells and immune cells. Blocking this protein can help boost the immune response against melanoma cells. This drug can be used along with the targeted drugs cobimetinib and vemurafenib in people with melanoma that has the BRAF gene mutation, when the cancer can't be removed by surgery or has spread to other parts of the body. This drug is given as an intravenous (IV) infusion, typically every 2 to 4 weeks.

CTLA-4 inhibitor - Ipilimumab (Yervoy) is another checkpoint inhibitor, but it has a different target. It blocks CTLA-4, another protein on T cells that normally helps keep them in check. It can be used to treat melanomas that can't be removed by surgery or that have spread to other parts of the body. It might also be used for less advanced melanomas after surgery (as an adjuvant treatment) in some situations, to try to lower the risk of the cancer coming back. When used alone, this drug is less likely to shrink tumors than the PD-1 inhibitors, and it tends to have more serious side effects, so usually one of those other drugs is tried first. Another option is to combine ipilimumab with one of the PD-1 inhibitors. This can increase the chance of shrinking the tumors (slightly more than a PD-1 inhibitor alone), but it can also increase the risk of side effects. This drug is given as an intravenous (IV) infusion, usually once every 3 weeks for 4 treatments (although it may be given for longer when used as an adjuvant treatment).

LAG-3 inhibitor - Relatlimab targets LAG-3, another checkpoint protein on certain immune cells that normally helps keep the immune system in check. This drug is given along with the PD-1 inhibitor nivolumab (in a combination known as Opdualag). It can be used to treat melanomas that can't be removed by surgery or that have spread to other parts of the body. This drug is given as an intravenous (IV) infusion, typically once every 4 weeks.

Therefore, as evidence is mounting that immune checkpoint inhibitors may at least benefit some patients there is an emerging need for reliable stratification of patients in groups who may benefits from checkpoint inhibitors and those who do not benefit. Herein the inventors have identified genes and gene signatures who have been validated on clinical datasets to reliably distinguish such patient groups.

The present predictions are obtained from public datasets TCGA and is described in Genomic Classification of Cutaneous Melanoma, Akbani, Rehan et al., Cell, Volume 161, Issue 7, 1681 - 1696, hereby incorporated by reference in its entirety.

Pembroluzimab is sold under the brand name Keytruda, is a humanized antibody, more specifically a PD-1 Inhibitor, used in cancer immunotherapy that treats melanoma, lung cancer, head and neck cancer, Hodgkin lymphoma, stomach cancer, cervical cancer, and certain types of breast cancer.

The protein sequences for the Pembroluzimab heavy and light chain proteins are provided below with SEQ ID Nos 167 and 168. The complementary determining regions (CDRs) of the heavy chain (HCCDR1-3) and the light chain (LCCDR1-3) are also listed below with SEQ ID Nos 169-174.

Pembrolizumab Heavy chain - SEQ ID NO: 167

QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LMISRTPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGK

Pembrolizumab Light chain - SEQ ID NO: 168

EIVLTQSPAT LSLSPGERAT LSCRASKGVS TSGYSYLHWY QQKPGQAPRL LIYLASYLES
GVPARFSGSG SGTDFTLTIS SLEPEDFAVY YCQHSRDLPL TFGGGTKVEI KRTVAAPSVF
IFPPSDEQLK SGTASVVCLL NNFYPREAKV QWKVDNALQS GNSQESVTEQ DSKDSTYSLS
STLTLSKADY EKHKVYACEV THQGLSSPVT KSFNRGEC

Complementary determining regions of Pembrolizumab

Pembrolizumab HCCDR1 (SEQ ID NO: 169): NYYMY
Pembrolizumab HCCDR2 (SEQ ID NO: 170): GINPSNGGTNFNEKFKN
Pembrolizumab HCCDR3 (SEQ ID NO: 171): RDYRFDMGFD
Pembrolizumab LCCDR1 (SEQ ID NO: 172): RASKGVSTSGYSYLHWY
Pembrolizumab LCCDR2 (SEQ ID NO: 173): LASYLES
Pembrolizumab LCCDR3 (SEQ ID NO: 174): QHSRDLPLT

When used herein the term "equivalent" when referring to Pembroluzimab refers to an antibody with substantially the same sequence as Pembroluzimab and which binds the same epitope. Thus the equivalent may comprise a heavy chain with at least 90%, preferably 91%, 92%, 93%, 94%, 95%, 96%, 96%, 97%, 98%, 99%, sequence identity to SEQ ID No 167 and comprises a light chain with at least 90%, preferably 91%, 92%, 93%, 94%, 95%, 96%, 96%, 97%, 98%, 99%, sequence identity to SEQ ID No 168. Preferably the equivalent comprises a heavy chain with at least 90%, preferably 91%, 92%, 93%, 94%, 95%, 96%, 96%, 97%, 98%, 99%, sequence identity to SEQ ID No 167 and comprises a light chain with at least 90%, preferably 91%, 92%, 93%, 94%, 95%, 96%, 96%, 97%, 98%, 99%, sequence identity to SEQ ID No 168 wherein the heavy chain CDRs are defined with SEQ ID Nos 169-171 and/or the light chain CDRs are defined with SEQ ID Nos 172-174. In a most preferred embodiment the equivalent comprises a heavy chain with at least 90%, preferably 91%, 92%, 93%, 94%, 95%, 96%, 96%, 97%, 98%, 99%, sequence identity to SEQ ID No 167 and comprises a light chain with at least 90%, preferably 91%, 92%, 93%, 94%, 95%, 96%, 96%, 97%, 98%, 99%, sequence identity to SEQ ID No 168 wherein the heavy chain CDRs are defined with SEQ ID Nos 169-171 and the light chain CDRs are defined with SEQ ID Nos 172-174.

The CDRs are indicated as follows: for the heavy chain CDR1 is amino acid 31-35 of SEQ ID NO: 169, CDR2 is amino acid 50-65 of SEQ ID NO: 169 and CDR3 is amino acid 99-102 of SEQ ID NO: 169. For the light chain CDR1 is amino acid 24-34 of SEQ ID NO: 170, CDR2 is amino acid 50-56 of SEQ ID NO: 170 and CDR3 is amino acid 89-97 of SEQ ID NO: 170. Thus when indicated above where for example a sequence has 90% sequence identity with SEQ ID NO: 167 and the CDRs are defined as SEQ ID NO: 169-171, it means that positions 31-35, 50-65, and 99-102 corresponding to SEQ ID NO:: 167 are defined with the sequences indicated in SEQ ID Nos 169-171 and the remaining sequence has identity such that at most 90% of the total sequence

In an embodiment the one or more gene expression levels, for example 1, 2, 3, 4, 5, 6, 7, 8, 9, 10, 11, 12, or more gene expression levels, are selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1, preferably selected from DHX9, PAG1, FYN, DDX58, and PRKACA. In an embodiment the one or more, for example 1, 2, 3, 4, 5, 6, 7, 8, 9, 10, 11, 12, or more gene expression levels further comprises one or more, for example 1, 2, 3, 4, 5, 6, 7or all 7, gene expression levels selected from CIAO1, MYD88, CSK, LAT, PDE4D, PRKACA and PRKACB. In an embodiment the prediction is based on six or more gene expression levels selected from AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, and ZAP70.

Thus in an embodiment the invention relates to Pembroluzimab or an equivalent thereof for use in the treatment of melanoma in a subject, the use comprising: determining or receiving the result of a determination of six or more gene, for example 6, 7, 8, 9, 10, 11, 12, 13, 14, 15, 16 or more, expression levels selected from AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, and ZAP70; said gene expression levels being determined in a biological sample obtained from the subject; and determining the prediction of the outcome based on six or more gene expression levels, wherein the outcome is a poor outcome or a good outcome, and when the predicted outcome is a poor outcome Pembroluzimab or an equivalent thereof is administered to the subject. Alternatively the invention relates to a method for treating a subject suffering from melanoma, the method comprising determining or receiving the result of a determination of six or more, for example 6, 7, 8, 9, 10, 11, 12, 13, 14, 15, 16 or more, gene expression levels selected from AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, and ZAP70; said gene expression levels being determined in a biological sample obtained from the subject; and determining the prediction of the outcome based on six or more gene expression levels, wherein the outcome is a poor outcome or a good outcome, and administering Pembroluzimab or an equivalent thereof to the subject when the predicted outcome is a poor outcome.

In an embodiment the use or method further comprises a step of providing the prediction to a medical caregiver or the subject.

In an embodiment the biological sample is obtained from the subject before the start of a therapy. In an embodiment the biological sample is a biopsy, preferably a biopsy from the tumor or a metastasis thereof, obtained from the subject, preferably obtained prior to the start of the therapy. Alternatively but not limiting the biological sample may be a blood sample including but not limited to a whole blood sample, a plasma sample, PBMCs or isolated blood cells, or the biological sample may be a tissue sample, a stool sample, a saliva sample, a bodily fluid sample, a urine sample, a cerebrospinal fluid sample, a interstitial fluid sample or a bile sample. Thus the term "biological sample" or "sample obtained from a subject" refers to any biological material obtained via suitable methods known to the person skilled in the art from a subject, e.g., a patient having a melanoma. The biological sample used may be collected in a clinically acceptable manner, e.g., in a way that nucleic acids (in particular RNA) or proteins are preserved. Preferably the biological sample contains cells, preferably tumor cells, or nucleic acids such as DNA or RNA, preferably nucleic acids derived from tumor cells.

The biological sample(s) may include body tissue and/or a fluid, such as, but not limited to, blood (or blood derived such as serum, plasma, or PBMC's (peripheral blood mononuclear cells)), sweat, saliva, urine, and a needle biopsy or resection biopsy. Furthermore, the biological sample may contain a cell extract derived from or a cell population including a cancerous cell or a cell derived from tissue suspected to be cancerous, such as a melanoma cell. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., melanoma cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

In an embodiment the invention relates to a method for treating a subject suffering from melanoma, the method comprising determining or receiving the result of a determination of six or more gene expression levels selected from AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, ZAP70; said gene expression levels being determined in a biological sample obtained from the subject; and determining the prediction of the outcome based on six or more gene expression levels, wherein the outcome is a poor outcome or a good outcome, and administering Pembroluzimab or an equivalent thereof to the subject when the predicted outcome is a poor outcome, or performing standard of care treatment when the predicted outcome is good. In an embodiment the standard of care includes radiotherapy, surgery, chemotherapy, targeted therapy, immune checkpoint inhibitor therapy, or combinations thereof. Non limiting examples of immune checkpoint inhibitors are are Ipilimumab (Yervoy), tremelimumab, Nivolumab (Opdivo), Pembrolizumab (Keytruda), Atezolizumab (Tecentriq), Avelumab (Bavencio), Durvalumab (Imfinzi), Cemiplimab (Libtayo), Dostarlimab (Jemperli), JTX-4014, Spartalizumab (PDR001), Camrelizumab (SHR1210), Sintilimab (IBI308), Tislelizumab (BGB-A317), Toripalimab (JS 001), Relatlimab (BMS-986016), INCMGA00012 (MGA012), AMP-224, AMP-514 (MEDI0680), KN035, CK-301, AUNP12, CA-170, BCD-100, and BMS-986189, more preferably an antibody treatment selected from Ipilimumab (Yervoy), tremelimumab, Nivolumab (Opdivo), Pembrolizumab (Keytruda), Atezolizumab (Tecentriq), Avelumab (Bavencio), Durvalumab (Imfinzi), Cemiplimab (Libtayo), Dostarlimab (Jemperli), JTX-4014, Spartalizumab (PDR001), Camrelizumab (SHR1210), Sintilimab (IBI308), Tislelizumab (BGB-A317), Toripalimab (JS 001), INCMGA00012 (MGA012), AMP-224, AMP-514 (MEDI0680), KN035, CK-301, AUNP12, CA-170, LY3300054, INCAGN02385, Sym023, Cobolimab (TSR-022), RO7121661, AZD7789, LY3321367, LB1410, INCAGN02390 and BMS-986189.

In a second aspect the invention relates to a method of predicting an optimal treatment strategy for a subject having melanoma, the method comprising: determining or receiving the result of a determination of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and determining the prediction of the outcome based on one or more gene expression levels, wherein the outcome is a poor outcome or a good outcome, and when the predicted outcome is a poor outcome the suggested treatment strategy comprises administering Pembroluzimab or an equivalent thereof. The method thus predicts an optimal treatment strategy for subjects with melanoma with a poor predicted outcome.

The genes tested herein are from the earlier described IDR_14 and TCR_17 gene signatures also described below.

**Immune** response defense genes

The integrity and stability of genomics DNA is permanently under stress induced by various cell internal and external factors like exposure to radiation, viral or bacterial infections, but also oxidation and replication stress (see Gasser S. et al., "Sensing of dangerous DNA", Mechanisms of Aging and Development, Vol. 165, pages 33-46, 2017). In order to maintain DNA structure and stability, a cell must be able to recognize all types of DNA damages like single or double strand breaks etc. induced by various factors. This process involves the participation of a multitude of specific proteins depending on the kind of damage as part of DNA recognition pathways.

Recent evidence suggests that mis-localized DNA (e.g., DNA unnaturally appearing in the cytosolic fraction of the cell in contrast to the nucleus) and damaged DNA (e.g., through mutations occurring in cancer development) is used by the immune system to identify infected or otherwise diseased cells while genomic and mitochondrial DNA present in healthy cells is ignored by DNA recognition pathways. In diseased cells, cytosolic DNA sensor proteins have been demonstrated to be involved in the detection of DNA occurring unnaturally in the cytosol of the cell. Detection of such DNA by different nucleic acid sensors translates into similar responses leading to nuclear factor kappa-B (NF-kB) and interferon type I (IFN type I) signalling followed by the activation of innate immune system components. While the recognition of viral DNA is known to induce an IFN type I response, evidence that sensing of DNA damage can initiate immune responses has only recently been accumulating.

TLR9 (Toll-like receptor 9) located in the endosomes was one of the first DNA sensors molecules identified to be involved in the immune recognition of DNA by signalling downstream via the adaptor protein myeloid differentiation primary-response protein88 (MYD88). This interaction in turn activates mitogen-activated protein kinases (MAPKs) and NF-kB. TLR9 also induces the generation of type I interferons through the activation of IRF7 via IkB Kinase alpha (IKKalpha) in plasmacytoid dendritic cells (pDCs). Various other DNA immune receptors including IFI16 (IFN-gamma-inducible protein 16), cGAS (cyclic DMP-AMP synthase, DDX41 (DEAD-box helicase 41), as well as ZBP1 (Z-DNA-binding protein 1) interact with STING (stimulator of IFN genes), which activates the IKK complex and IRF3 through TBK1 (TANK binding kinase 1). ZBP1 also activates NF-kB via recruitment of RIP1 and RIP3 (receptor-interacting protein 1 and 3, respectively). While the helicase DHX36 (DEAH-box helicase 36) interacts in a complex with TRID to induce NF-kB and IRF-3/7 the DHX9 helicase stimulates MYD88-dependent signalling in plasmacytoid dendritic cells. The DNA sensor LRRFIP1 (leucine-rich repeat flightless-interacting protein) complexes with beta-catenin to activate the transcription of IRF3 whereas AIM2 (absent in melanoma 2) recruits the adaptor protein ASC (apoptosis speck-like protein) to induce a caspase-1-activating inflammasome complex leading to the secretion of interleukin-1beta (IL-1beta) and IL-18 (see Fig. 1 of Gasser S. et al., 2017, ibid, which provides a schematic overview of DNA damage and DNA sensor pathways leading to the production of inflammatory cytokines and the expression of ligands for activating innate immune receptors. Members of the non-homologous end joining pathway (orange), homologous recombination (red), inflammasome (dark green), NF-kB and interferon responses (light green) are shown).

The factors and mechanisms responsible for activating the DNA sensor pathways in cancer are currently not well elucidated. It will be important to identify the intratumoral DNA species, sensors and pathways implicated in the expression of IFNs in different cancer types at all stages of the disease. In addition to therapeutic targets in cancer, such factors may also have prognostic and predictive value. Novel DNA sensor pathway agonists and antagonists are currently being developed and tested in preclinical trials. Such compounds will be useful in characterizing the role of DNA sensor pathways in the pathogenesis of cancer, autoimmunity and potentially other diseases.

**T-Cell receptor signaling genes**

An immune response against pathogens can be elicited at different levels: there are physical barriers, such as the skin, to keep invaders out. If breached, innate immunity comes into play; a first and fast non-specific response. If this is not sufficient, the adaptive immune response is elicited. This is much more specific and needs time to develop when encountering a pathogen for the first time. Lymphocytes are activated by interacting with activated antigen presenting cells from the innate immune system, and are also responsible for maintenance of memory for faster responses upon next encounters with the same pathogen.

As lymphocytes are highly specific and effective when activated, they are subject to negative selection for their ability to recognize self, a process known as central tolerance. As not all self-antigens are expressed at selection sites, peripheral tolerance mechanisms evolved as well, such as ligation of the TCR in absence of co-stimulation, expression of inhibitory co-receptors, and suppression by Tregs. A disturbed balance between activation and suppression may lead to autoimmune disorders, or immune deficiencies and cancer, respectively.

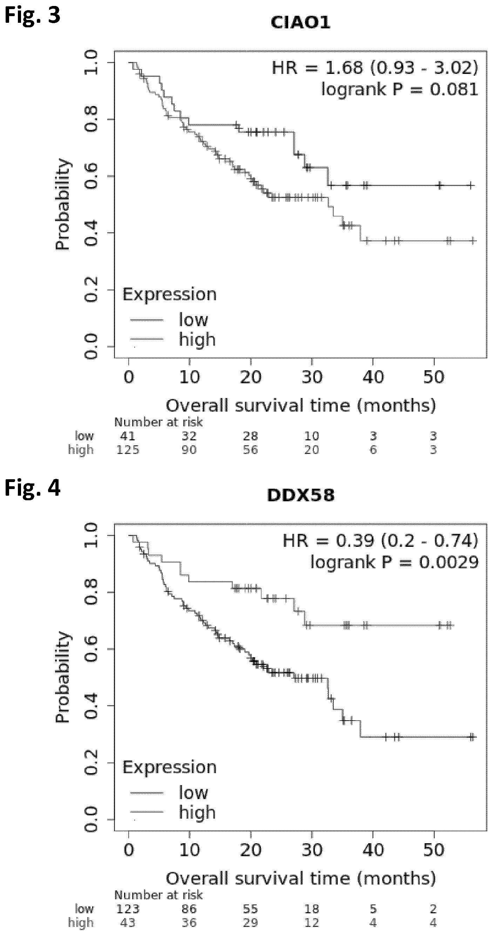

T-cell activation can have different functional consequences, depending on the location the type of T-cell involved. CD8+ T-cells differentiate into cytotoxic effector cells, whereas CD4+ T-cells can differentiate into Th1 (IFNγ secretion and promotion of cell mediated immunity) or Th2 (IL4/5/13 secretion and promotion of B cell and humoral immunity). Differentiation towards other, more recently identified T-cell subsets is also possible, for example the Tregs, which have a suppressive effect on immune activation (see Mosenden R. and Tasken K., "Cyclic AMP-mediated immune regulation - Overview of mechanisms of action in T-cells", Cell Signal, Vol. 23, No. 6, pages 1009-1016, 2011, in particular, Fig. 4, which T-cell activation and its modulation by PKA, and Tasken K. and Ruppelt A., "Negative regulation of T-cell receptor activation by the cAMP-PKA-Csk signaling pathway in T-cell lipid rafts", Front Biosci, Vol. 11, pages 2929-2939, 2006).

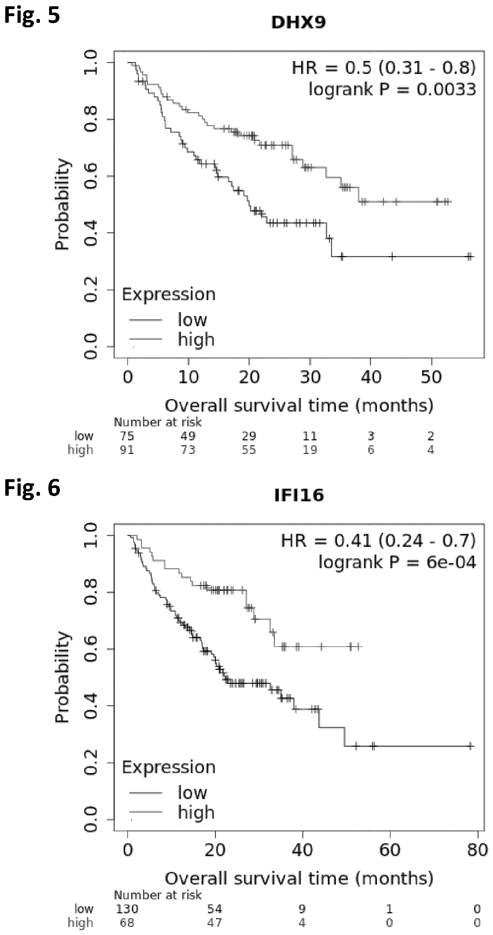

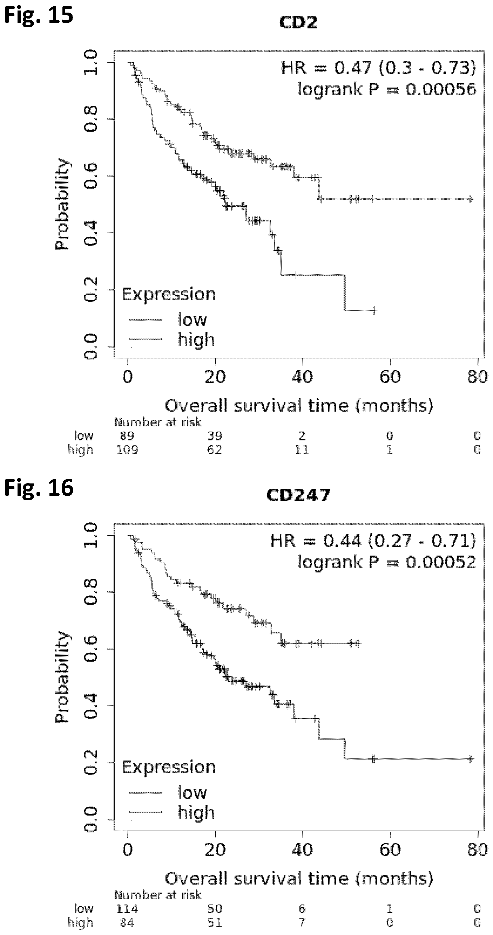

Both PKA and PDE4 regulated signaling intersect with TCR induced T-cell activation to fine-tune its regulation, with opposing effects (see Abrahamsen H. et al., "TCR- and CD28-mediated recruitment of phosphodiesterase 4 to lipid rafts potentiates TCR signaling", J Immunol, Vol. 173, pages 4847-4848, 2004, in particular, Fig. 6, which shows opposing effects of PKA and PDE4 on TCR activation). The molecule that connects these effectors is cyclic AMP (cAMP), an intracellular second messenger of extracellular ligand action. In T-cells, it mediates effects of prostaglandins, adenosine, histamine, beta-adrenergic agonists, neuropeptide hormones and beta-endorphin. Binding of these extracellular molecules to GPCRs leads to their conformational change, release of stimulatory subunits and subsequent activation of adenylate cyclases (AC), which hydrolyze ATP to cAMP (see Fig. 6 of Abrahamsen H. et al., 2004, ibid). Although not the only one, PKA is the principal effector of cAMP signaling (see Mosenden R. and Tasken K., 2011, ibid, and Tasken K. and Ruppelt A., 2006, ibid). At a functional level, increased levels of cAMP lead to reduced IFNγ and IL-2 production in T-cells (see Abrahamsen H. et al., 2004, ibid). Aside from interfering with TCR activation, PKA has many more effector (see Fig. 15 of Torheim E.A., "Immunity Leashed - Mechanisms of Regulation in the Human Immune System", Thesis for the degree of Philosophiae Doctor (PhD), The Biotechnology Centre of Ola, University of Oslo, Norway, 2009).

In naive T-cells, hyperphosphorylated PAG targets Csk to lipid rafts. Via the Ezrin-EBP50-PAG scaffold complex PKA is targeted to Csk. Through specific phosphorylation by PKA, Csk can negatively regulate Lck and Fyn to dampen their activity and downregulate T-cell activation (see Fig. 6 of Abrahamsen H. et al., 2004, ibid). Upon TCR activation, PAG is dephosphorylated and Csk is released from the rafts. Dissociation of Csk is needed for T-cell activation to proceed. Within the same time course, a Csk-G3BP complex is formed and seems to sequester Csk outside lipid rafts (see Mosenden R. and Tasken K., 2011, ibid, and Tasken K. and Ruppelt A., 2006, ibid).

In contrast, combined TCR and CD28 stimulation mediates recruitment of the cyclic nucleotide phosphodiesterase PDE4 to lipid rafts, which enhances cAMP degradation (see Fig. 6 of Abrahamsen H. et al., 2004, ibid). As such, TCR induced production of cAMP is countered, and the T-cell immune response potentiated. Upon TCR stimulation alone, PDE4 recruitment may be too low to fully reduce the cAMP levels and therefore maximal T-cell activation cannot occur (see Abrahamsen H. et al., 2004, ibid).

Thus, by active suppression of proximal TCR signaling, signaling via cAMP-PKA-Csk is thought to set the threshold for T-cell activation. Recruitment of PDEs can counter this suppression. Tissue or cell-type specific regulation is accomplished through expression of multiple isoforms of AC, PKA, and PDEs. As mentioned above, the balance between activation and suppression needs to be tightly regulated to prevent development of autoimmune disorders, immune deficiencies and cancer.

**Selection of the genes**

The lists of genes have originally been selected by us for prognostication in prostate cancer subjects. In this document, it is shown that they are also of prognostic value with respect to an outcome of a subject having a melanoma.

The identified immune defense response genes ZBP1, and AIM2, APOBEC3A, CIAO1, DDX58, DHX9, IFI16, IFIH1, IFIT1, IFIT3, LRRFIP1, MYD88, OAS1, TLR8, and ZBP1, respectively, were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #2 demonstrated enrichment (enrichment score: 10.8) in 30 genes with a function in defense response to viruses, negative regulation of viral genome replication as well as type I interferon signaling. A further heat map analysis confirmed that these immune defense response genes were generally higher expressed in samples from patients in PDE4D7 score class 1 than from patients in PDE4D7 score class 4. The class of genes with a function in defense response to viruses, negative regulation of viral genome replication as well as type I interferon signaling was further enriched to 61 genes by literature search to identify additional genes with the same molecular function. A further selection from the 61 genes was made based on the combinatorial power to separate patients who died from prostate cancer vs. those who did not, resulting of a preferred set of 14 genes. It was found that the number of events (metastases, prostate cancer specific death) was enriched in sub-cohorts with a low expression of these genes compared to the total patient cohort (#538) and a sub-cohort of 151 patients undergoing salvage RT (SRT) after post-surgical disease recurrence.

The identified T-Cell receptor signaling genes CD2, CD247, CD28, CD3E, CD3G, CD4, CSK, EZR, FYN, LAT, LCK, PAG1, PDE4D, PRKACA, PRKACB, PTPRC, and ZAP70 were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #6 demonstrated enrichment (enrichment score: 5.9) in 17 genes with a function in primary immune deficiency and activation of T-Cell receptor signaling. A further heat map analysis confirmed that these T-Cell receptor signaling genes were generally higher expressed in samples from patients in PDE4D7 score class 1 than from patients in PDE4D7 score class 4.

In this document, it is shown that these genes are also of prognostic value with respect to an outcome of a subject suffering from a melanoma. Herein the inventors describe data obtained from different melanoma patients that the gene signatures "Immune defense response genes", and "T-cell receptor signaling genes" each individually or their combination (SKCMAI score) could stratify patients based on the end point overall death (survival time in months).

With respect to the biological processes described above, two immune system related gene signatures were selected, including the genes listed in tables 1 and 2. Before, the relevance of these signatures to prediction of prostate cancer survival was shown before.

Table 1: Immune Defence Response (IDR) Signature.

| Gene Symbol | Ensembl_ID |
|---|---|
| AIM2 | ENSG00000163568 |
| APOBEC3A | ENSG00000128383 |
| CIAO1 | ENSG00000144021 |
| DDX58 | ENSG00000107201 |
| DHX9 | ENSG00000135829 |
| IFI16 | ENSG00000163565 |
| IFIH1 | ENSG00000115267 |
| IFIT1 | ENSG00000185745 |
| IFIT3 | ENSG00000119917 |
| LRRFIP1 | ENSG00000124831 |
| MYD88 | ENSG00000172936 |
| OAS1 | ENSG00000089127 |
| TLR8 | ENSG00000101916 |
| ZBP1 | ENSG00000124256 |

Table 2: T-Cell Receptor (TCR) Signature.

| Gene Symbol | Ensembl _ID |
|---|---|
| CD2 | ENSG00000116824 |
| CD247 | ENSG00000198821 |
| CD28 | ENSG00000178562 |
| CD3E | ENSG00000198851 |
| CD3G | ENSG00000160654 |
| CD4 | ENSG00000010610 |
| CSK | ENSG00000103653 |
| EZR | ENSG00000092820 |
| FYN | ENSG00000010810 |
| LAT | ENSG00000213658 |
| LCK | ENSG00000182866 |
| PAG1 | ENSG00000076641 |
| PDE4D | ENSG00000113448 |
| PRKACA | ENSG00000072062 |
| PRKACB | ENSG00000142875 |

(continued)

| Gene Symbol | Ensembl _ID |
|-------------|-----------------|
| PTPRC | ENSG00000081237 |
| ZAP70 | ENSG00000115085 |

It was found that the genes AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, and ZAP70 each individually, one or more per gene panel, in a combination of one or more per gene panel or when all combined, are able to predict the outcome in a subject having a melanoma.

The term "ABCC5" refers to the human ATP binding cassette subfamily C member 5 gene (Ensembl: ENSG00000114770), for example, to the sequence as defined in NCBI Reference Sequence NM_001023587.2 or in NCBI Reference Sequence NM_005688.3, specifically, to the nucleotide sequence as set forth in SEQ ID NO:1 or in SEQ ID NO:2, which correspond to the sequences of the above indicated NCBI Reference Sequences of the ABCC5 transcript, and also relates to the corresponding amino acid sequence for example as set forth in SEQ ID NO:3 or in SEQ ID NO:4, which correspond to the protein sequences defined in NCBI Protein Accession Reference Sequence NP_001018881.1 and in NCBI Protein Accession Reference Sequence NP_005679 encoding the ABCC5 polypeptide.

The term "ABCC5" also comprises nucleotide sequences showing a high degree of homology to ABCC5, e.g., nucleic acid sequences being at least 75%, 80%, 85%, 90%, 91%, 92%, 93%, 94%, 95%, 96%, 97%, 98% or 99% identical to the sequence as set forth in SEQ ID NO:1 or in SEQ ID NO:2 or amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1 or in SEQ ID NO:2.

The term "AIM2" refers to the Absent in Melanoma 2 gene (Ensembl: ENSG00000163568), for example, to the sequence as defined in NCBI Reference Sequence NM_004833, specifically, to the nucleotide sequence as set forth in SEQ ID NO:5, which corresponds to the sequence of the above indicated NCBI Reference Sequence of the AIM2 transcript, and also relates to the corresponding amino acid sequence for example as set forth in SEQ ID NO:6, which corresponds to the protein sequence defined in NCBI Protein Accession Reference Sequence NP_004824 encoding the AIM2 polypeptide.

The term "AIM2" also comprises nucleotide sequences showing a high degree of homology to AIM2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "APOBEC3A" refers to the Apolipoprotein B mRNA Editing Enzyme Catalytic Subunit 3A gene (Ensembl: ENSG00000128383), for example, to the sequence as defined in NCBI Reference Sequence NM_145699, specifically, to the nucleotide sequence as set forth in SEQ ID NO:7, which corresponds to the sequence of the above indicated NCBI Reference Sequence of the APOBEC3A transcript, and also relates to the corresponding amino acid sequence for example as set forth in SEQ ID NO:8, which corresponds to the protein sequence defined in NCBI Protein Accession Reference Sequence NP_663745 encoding the APOBEC3A polypeptide.

The term "APOBEC3A" also comprises nucleotide sequences showing a high degree of homology to APOBEC3A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:7 or amino acid sequences being at least 75%, 80%, 85%, 90%, 91%, 92%, 93%, 94%, 95%, 96%, 97%, 98% or 99% identical to the sequence as set forth in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7.

The term "CD2" refers to the Cluster Of Differentiation 2 gene (Ensembl: ENSG00000116824), for example, to the sequence as defined in NCBI Reference Sequence NM_001767, specifically, to the nucleotide sequence as set forth in SEQ ID NO:9, which corresponds to the sequence of the above indicated NCBI Reference Sequence of the CD2 transcript, and also relates to the corresponding amino acid sequence for example as set forth in SEQ ID NO: 10, which corresponds to the protein sequence defined in NCBI Protein Accession Reference Sequence NP_001758 encoding the CD2 polypeptide.

The term "CD2" also comprises nucleotide sequences showing a high degree of homology to CD2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO: 10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 10 or amino acid sequences being encoded by nucleic acid sequences being at least 75%, 80%, 85%, 90%, 91% 92%, 93%, 94%, 95%, 96%, 97%, 98% or 99% identical to the sequence as set forth in SEQ ID NO:9.

The term "CD247" refers to the Cluster Of Differentiation 247 gene (Ensembl: ENSG00000198821), for example, to the sequence as defined in NCBI Reference Sequence NM_000734 or in NCBI Reference Sequence NM_198053, specifically, to the nucleotide sequence as set forth in SEQ ID NO:11 or in SEQ ID NO:12, which correspond to the sequences of the above indicated NCBI Reference Sequences of the CD247 transcript, and also relates to the corresponding amino acid sequence for example as set forth in SEQ ID NO:13 or in SEQ ID NO:14, which correspond to the protein sequences defined in NCBI Protein Accession Reference Sequence NP_000725 and in NCBI Protein Accession Reference Sequence NP_932170 encoding the CD247 polypeptide.

The term "CD247" also comprises nucleotide sequences showing a high degree of homology to CD247, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 11 or in SEQ ID NO: 12 or amino acid sequences being at least 75%, 80%, 85%, 90%, 91%, 92%, 93%, 94%, 95%, 96%, 97%, 98% or 99% identical to the sequence as set forth in SEQ ID NO: 13 or in SEQ ID NO: 14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 13 or in SEQ ID NO: 14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12.

The term "CD28" refers to the Cluster Of Differentiation 28 gene (Ensembl: ENSG00000178562), for example, to the sequence as defined in NCBI Reference Sequence NM_006139 or in NCBI Reference Sequence NM_001243078, specifically, to the nucleotide sequence as set forth in SEQ ID NO: 15 or in SEQ ID NO: 16, which correspond to the sequences of the above indicated NCBI Reference Sequences of the CD28 transcript, and also relates to the corresponding amino acid sequence for example as set forth in SEQ ID NO: 17 or in SEQ ID NO: 18, which correspond to the protein sequences defined in NCBI Protein Accession Reference Sequence NP_006130 and in NCBI Protein Accession Reference Sequence NP_001230007 encoding the CD28 polypeptide.

The term "CD28" also comprises nucleotide sequences showing a high degree of homology to CD28, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO: 16 or amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO: 18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO: 18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO: 16.

The term "CD3E" refers to the Cluster Of Differentiation 3E gene (Ensembl: ENSG00000198851), for example, to the sequence as defined in NCBI Reference Sequence NM_000733, specifically, to the nucleotide sequence as set forth in SEQ ID NO: 19, which corresponds to the sequence of the above indicated NCBI Reference Sequence of the CD3E transcript, and also relates to the corresponding amino acid sequence for example as set forth in SEQ ID NO:20, which corresponds to the protein sequence defined in NCBI Protein Accession Reference Sequence NP_000724 encoding the CD3E polypeptide.

The term "CD3E" also comprises nucleotide sequences showing a high degree of homology to CD3E, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 19.

The term "CD3G" refers to the Cluster Of Differentiation 3G gene (Ensembl: ENSG00000160654), for example, to the sequence as defined in NCBI Reference Sequence NM_000073, specifically, to the nucleotide sequence as set forth in SEQ ID NO:21, which corresponds to the sequence of the above indicated NCBI Reference Sequence of the CD3G transcript, and also relates to the corresponding amino acid sequence for example as set forth in SEQ ID NO:22, which corresponds to the protein sequence defined in NCBI Protein Accession Reference Sequence NP_000064 encoding the CD3G polypeptide.

The term "CD3G" also comprises nucleotide sequences showing a high degree of homology to CD3G, e.g., nucleic acid sequences being at least 75%, 80%, 85%, 90%, 91%, 92%, 93%, 94%, 95%, 96%, 97%, 98% or 99% identical to the sequence as set forth in SEQ ID NO:21 or amino acid sequences being at least 75%, 80%, 85%, 90%, 91%, 92%, 93%, 94%, 95%, 96%, 97%, 98% or 99% identical to the sequence as set forth in SEQ ID NO:22 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:22 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21.

The term "CD4" refers to the Cluster Of Differentiation 4 gene (Ensembl: ENSG00000010610), for example, to the sequence as defined in NCBI Reference Sequence NM_000616, specifically, to the nucleotide sequence as set forth in SEQ ID NO:23, which corresponds to the sequence of the above indicated NCBI Reference Sequence of the CD4 transcript, and also relates to the corresponding amino acid sequence for example as set forth in SEQ ID NO:24, which corresponds to the protein sequence defined in NCBI Protein Accession Reference Sequence NP_000607 encoding the CD4 polypeptide.

The term "CD4" also comprises nucleotide sequences showing a high degree of homology to CD4, e.g., nucleic acid sequences being at least 75%, 80%, 85%, 90%, 91%, 92%, 93%, 94%, 95%, 96%, 97%, 98% or 99% identical to the sequence as set forth in SEQ ID NO:23 or amino acid sequences being at least 75%, 80%, 85%, 90%, 91%, 92%, 93%, 94%, 95%, 96%, 97%, 98% or 99% identical to the sequence as set forth in SEQ ID NO:24 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:24 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:23.

The term "CIAO1" refers to the Cytosolic Iron-Sulfur Assembly Component 1 gene (Ensembl: ENSG00000144021), for example, to the sequence as defined in NCBI Reference Sequence NM_004804, specifically, to the nucleotide sequence as set forth in SEQ ID NO:25, which corresponds to the sequence of the above indicated NCBI Reference Sequence of the CIAO1 transcript, and also relates to the corresponding amino acid sequence for example as set forth in SEQ ID NO:26, which corresponds to the protein sequence defined in NCBI Protein Accession Reference Sequence NP_663745 encoding the CIAO1 polypeptide.

The term "CIAO1" also comprises nucleotide sequences showing a high degree of homology to CIAO1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:25 or amino acid sequences being at least 75%, 80%, 85%, 90%, 91%, 92%, 93%, 94%, 95%, 96%, 97%, 98% or 99% identical to the sequence as set forth in SEQ ID NO:26 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:26 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:25.

The term "CSK" refers to the C-Terminal Src Kinase gene (Ensembl: ENSG00000103653), for example, to the sequence as defined in NCBI Reference Sequence NM_004383, specifically, to the nucleotide sequence as set forth in SEQ ID NO:27, which corresponds to the sequence of the above indicated NCBI Reference Sequence of the CSK transcript, and also relates to the corresponding amino acid sequence for example as set forth in SEQ ID NO:28, which corresponds to the protein sequence defined in NCBI Protein Accession Reference Sequence NP_004374 encoding the CSK polypeptide.

The term "CSK" also comprises nucleotide sequences showing a high degree of homology to CSK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:27 or amino acid sequences being at least 75%, 80%, 85%, 90%, 91%, 92%, 93%, 94%, 95%, 96%, 97%, 98% or 99% identical to the sequence as set forth in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:27.

The term "CUX2" refers to the human Cut Like Homeobox 2 gene (Ensembl: ENSG00000111249), for example, to the sequence as defined in NCBI Reference Sequence NM_015267.3, specifically, to the nucleotide sequence as set forth in SEQ ID NO:29, which corresponds to the sequence of the above indicated NCBI Reference Sequence of the CUX2 transcript, and also relates to the corresponding amino acid sequence for example as set forth in SEQ ID NO:30, which corresponds to the protein sequence defined in NCBI Protein Accession Reference Sequence NP_056082.2 encoding the CUX2 polypeptide.

The term "CUX2" also comprises nucleotide sequences showing a high degree of homology to CUX2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:29 or amino acid sequences being at least 75%, 80%, 85%, 90%, 91%, 92%, 93%, 94%, 95%, 96%, 97%, 98% or 99% identical to the sequence as set forth in SEQ ID NO:30 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:30 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29.

The term "DDX58" refers to the DExD/H-box Helicase 58 gene (Ensembl: ENSG00000107201), for example, to the sequence as defined in NCBI Reference Sequence NM_014314, specifically, to the nucleotide sequence as set forth in SEQ ID NO:31, which corresponds to the sequence of the above indicated NCBI Reference Sequence of the DDX58 transcript, and also relates to the corresponding amino acid sequence for example as set forth in SEQ ID NO:32, which corresponds to the protein sequence defined in NCBI Protein Accession Reference Sequence NP_055129 encoding the DDX58 polypeptide.

The term "DDX58" also comprises nucleotide sequences showing a high degree of homology to DDX58, e.g., nucleic acid sequences being at least 75%, 80%, 85%, 90%, 91%, 92%, 93%, 94%, 95%, 96%, 97%, 98% or 99% identical to the sequence as set forth in SEQ ID NO:31 or amino acid sequences being at least 75%, 80%, 85%, 90%, 91%, 92%, 93%, 94%, 95%, 96%, 97%, 98% or 99% identical to the sequence as set forth in SEQ ID NO:32 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:32 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:31.

The term "DHX9" refers to the DExD/H-box Helicase 9 gene (Ensembl: ENSG00000135829), for example, to the sequence as defined in NCBI Reference Sequence NM_001357, specifically, to the nucleotide sequence as set forth in SEQ ID NO:33, which corresponds to the sequence of the above indicated NCBI Reference Sequence of the DHX9 transcript, and also relates to the corresponding amino acid sequence for example as set forth in SEQ ID NO:34, which corresponds to the protein sequence defined in NCBI Protein Accession Reference Sequence NP_001348 encoding the DHX9 polypeptide.

The term "DHX9" also comprises nucleotide sequences showing a high degree of homology to DHX9, e.g., nucleic acid sequences being at least 75%, 80%, 85%, 90%, 91%, 92%, 93%, 94%, 95%, 96%, 97%, 98% or 99% identical to the sequence as set forth in SEQ ID NO:33 or amino acid sequences being at least 75%, 80%, 85%, 90%, 91%, 92%, 93%, 94%, 95%, 96%, 97%, 98% or 99% identical to the sequence as set forth in SEQ ID NO:34 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:34 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:33.

The term "EZR" refers to the Ezrin gene (Ensembl: ENSG00000092820), for example, to the sequence as defined in NCBI Reference Sequence NM_003379, specifically, to the nucleotide sequence as set forth in SEQ ID NO:35, which corresponds to the sequence of the above indicated NCBI Reference Sequence of the EZR transcript, and also relates to the corresponding amino acid sequence for example as set forth in SEQ ID NO:36, which corresponds to the protein sequence defined in NCBI Protein Accession Reference Sequence NP_003370 encoding the EZR polypeptide.

The term "EZR" also comprises nucleotide sequences showing a high degree of homology to EZR, e.g., nucleic acid sequences being at least 75%, 80%, 85%, 90%, 91%, 92%, 93%, 94%, 95%, 96%, 97%, 98% or 99% identical to the sequence as set forth in SEQ ID NO:35 or amino acid sequences being at least 75%, 80%, 85%, 90%, 91%, 92%, 93%, 94%, 95%, 96%, 97%, 98% or 99% identical to the sequence as set forth in SEQ ID NO:36 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:36 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:35.

The term "FYN" refers to the FYN Proto-Oncogene gene (Ensembl: ENSG00000010810), for example, to the sequence as defined in NCBI Reference Sequence NM_002037 or in NCBI Reference Sequence NM_153047 or in NCBI Reference Sequence NM_153048, specifically, to the nucleotide sequence as set forth in SEQ ID NO:37 or in SEQ ID NO:38 or in SEQ ID NO:39, which correspond to the sequences of the above indicated NCBI Reference Sequences of the FYN transcript, and also relates to the corresponding amino acid sequence for example as set forth in SEQ ID NO:40 or in SEQ ID NO:41 or in SEQ ID NO:42, which correspond to the protein sequences defined in NCBI Protein Accession Reference Sequence NP_002028 and in NCBI Protein Accession Reference Sequence NP_694592 and in NCBI Protein Accession Reference Sequence XP_005266949 encoding the FYN polypeptide.

The term "FYN" also comprises nucleotide sequences showing a high degree of homology to FYN, e.g., nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39 or amino acid sequences being at least 75%, 80%, 85%, 90%, 91%, 92%, 93%, 94%, 95%, 96%, 97%, 98% or 99% identical to the sequence as set forth in SEQ ID NO:40 or in SEQ ID NO:41 or in SEQ ID NO:42 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:40 or in SEQ ID NO:41 or in SEQ ID NO:42 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39.

The term "IFI16" refers to the Interferon Gamma Inducible Protein 16 gene (Ensembl: ENSG00000163565), for example, to the sequence as defined in NCBI Reference Sequence NM_005531, specifically, to the nucleotide sequence as set forth in SEQ ID NO:43, which corresponds to the sequence of the above indicated NCBI Reference Sequence of the IFI16 transcript, and also relates to the corresponding amino acid sequence for example as set forth in SEQ ID NO:44, which corresponds to the protein sequence defined in NCBI Protein Accession Reference Sequence NP_005522 encoding the IFI16 polypeptide.

The term "IFI16" also comprises nucleotide sequences showing a high degree of homology to IFI16, e.g., nucleic acid sequences being at least 75%, 80%, 85%, 90%, 91%, 92%, 93%, 94%, 95%, 96%, 97%, 98% or 99% identical to the sequence as set forth in SEQ ID NO:43 or amino acid sequences being at least 75%, 80%, 85%, 90%, 91%, 92%, 93%, 94%, 95%, 96%, 97%, 98% or 99% identical to the sequence as set forth in SEQ ID NO:44 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:44 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:43.

The term "IFIH1" refers to the Interferon Induced With Helicase C Domain 1 gene (Ensembl: ENSG00000115267), for example, to the sequence as defined in NCBI Reference Sequence NM_022168, specifically, to the nucleotide sequence as set forth in SEQ ID NO:45, which corresponds to the sequence of the above indicated NCBI Reference Sequence of the IFIH1 transcript, and also relates to the corresponding amino acid sequence for example as set forth in SEQ ID NO:46, which corresponds to the protein sequence defined in NCBI Protein Accession Reference Sequence NP_071451 encoding the IFIH1 polypeptide.

The term "IFIH1" also comprises nucleotide sequences showing a high degree of homology to IFIH1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:45 or amino acid sequences being at least 75%, 80%, 85%, 90%, 91%, 92%, 93%, 94%, 95%, 96%, 97%, 98% or 99% identical to the sequence as set forth in SEQ ID NO:46 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:46 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:45.

The term "IFIT1" refers to the Interferon Induced Protein With Tetratricopeptide Repeats 1 gene (Ensembl: ENSG00000185745), for example, to the sequence as defined in NCBI Reference Sequence NM_001270929 or in NCBI Reference Sequence NM_001548.5, specifically, to the nucleotide sequence as set forth in SEQ ID NO:47 or in SEQ ID NO:48, which correspond to the sequences of the above indicated NCBI Reference Sequences of the IFIT1 transcript, and also relates to the corresponding amino acid sequence for example as set forth in SEQ ID NO:49 or in SEQ ID NO:50, which correspond to the protein sequences defined in NCBI Protein Accession Reference Sequence NP_001257858 and in NCBI Protein Accession Reference Sequence NP_001539 encoding the IFIT1 polypeptide.

The term "IFIT1" also comprises nucleotide sequences showing a high degree of homology to IFIT1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48 or amino acid sequences being at least 75%, 80%, 85%, 90%, 91%, 92%, 93%, 94%, 95%, 96%, 97%, 98% or 99% identical to the sequence as set forth in SEQ ID NO:49 or in SEQ ID NO:50 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:49 or SEQ ID NO:50 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48.

The term "IFIT3" refers to the Interferon Induced Protein With Tetratricopeptide Repeats 3 gene (Ensembl: ENSG00000119917), for example, to the sequence as defined in NCBI Reference Sequence NM_001031683, specifically, to the nucleotide sequence as set forth in SEQ ID NO:51, which corresponds to the sequence of the above indicated NCBI Reference Sequence of the IFIT3 transcript, and also relates to the corresponding amino acid sequence for example as set forth in SEQ ID NO:52, which corresponds to the protein sequence defined in NCBI Protein Accession Reference Sequence NP_001026853 encoding the IFIT3 polypeptide.

The term "IFIT3" also comprises nucleotide sequences showing a high degree of homology to IFIT3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:51 or amino acid sequences being at least 75%, 80%, 85%, 90%, 91%, 92%, 93%, 94%, 95%, 96%, 97%, 98% or 99% identical to the sequence as set forth in SEQ ID NO:52 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:52 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:51.

The term "KIAA1549" refers to the human KIAA1549 gene (Ensembl: ENSG00000122778), for example, to the sequence as defined in NCBI Reference Sequence NM_020910 or in NCBI Reference Sequence NM_001164665, specifically, to the nucleotide sequence as set forth in SEQ ID NO:53 or in SEQ ID NO:54, which correspond to the sequences of the above indicated NCBI Reference Sequence of the KIAA1549 transcript, and also relates to the corresponding amino acid sequence for example as set forth in SEQ ID NO:55 or in SEQ ID NO:56, which correspond to the protein sequences defined in NCBI Protein Accession Reference Sequence NP_065961 and in NCBI Protein Accession Reference Sequence NP_001158137 encoding the KIAA1549 polypeptide.

The term "KIAA1549" also comprises nucleotide sequences showing a high degree of homology to KIAA1549, e.g., nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54 or amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54.

The term "LAT" refers to the Linker For Activation Of T-Cells gene (Ensembl: ENSG00000213658), for example, to the sequence as defined in NCBI Reference Sequence NM_001014987 or in NCBI Reference Sequence NM_014387, specifically, to the nucleotide sequence as set forth in SEQ ID NO:57 or in SEQ ID NO:58, which correspond to the sequences of the above indicated NCBI Reference Sequences of the LAT transcript, and also relates to the corresponding amino acid sequence for example as set forth in SEQ ID NO:59 or in SEQ ID NO:60, which corresponds to the protein sequences defined in NCBI Protein Accession Reference Sequence NP_001014987 and in NCBI Protein Accession Reference Sequence NP_055202 encoding the LAT polypeptide.

The term "LAT" also comprises nucleotide sequences showing a high degree of homology to LAT, e.g., nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58 or amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58.

The term "LCK" refers to the LCK Proto-Oncogene gene (Ensembl: ENSG00000182866), for example, to the sequence as defined in NCBI Reference Sequence NM_005356, specifically, to the nucleotide sequence as set forth in SEQ ID NO:61, which corresponds to the sequence of the above indicated NCBI Reference Sequence of the LCK transcript, and also relates to the corresponding amino acid sequence for example as set forth in SEQ ID NO:62, which corresponds to the protein sequence defined in NCBI Protein Accession Reference Sequence NP_005347 encoding the LCK polypeptide.

The term "LCK" also comprises nucleotide sequences showing a high degree of homology to LCK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:61 or amino acid sequences being at least 75%, 80%, 85%, 90%, 91%, 92%, 93%, 94%, 95%, 96%, 97%, 98% or 99% identical to the sequence as set forth in SEQ ID NO:62 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:62 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:61.

The term "LRRFIP1" refers to the LRR Binding FLII Interacting Protein 1 gene (Ensembl: ENSG00000124831), for example, to the sequence as defined in NCBI Reference Sequence NM_004735 or in NCBI Reference Sequence NM_001137550 or in NCBI Reference Sequence NM_001137553 or in NCBI Reference Sequence NM_001137552, specifically, to the nucleotide sequence as set forth in SEQ ID NO:63 or in SEQ ID NO:64 or in SEQ ID NO:65 or in SEQ ID NO:66, which correspond to the sequences of the above indicated NCBI Reference Sequences of the LRRFIP1 transcript, and also relates to the corresponding amino acid sequence for example as set forth in SEQ ID NO:67 or in SEQ ID NO:68 or in SEQ ID NO:69 or in SEQ ID NO:70, which correspond to the protein sequences defined in NCBI Protein Accession Reference Sequence NP_004726 and in NCBI Protein Accession Reference Sequence NP_001131022 and in NCBI Protein Accession Reference Sequence NP_001131025 and in NCBI Protein Accession Reference Sequence NP_001131024 encoding the LRRFIP1 polypeptide.

The term "LRRFIP1" also comprises nucleotide sequences showing a high degree of homology to LRRFIP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66 or amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66.

The term "MYD88" refers to the MYD88 Innate Immune Signal Transduction Adaptor gene (Ensembl: ENSG00000172936), for example, to the sequence as defined in NCBI Reference Sequence NM_001172567 or in NCBI Reference Sequence NM_001172568 or in NCBI Reference Sequence NM_001172569 or in NCBI Reference Sequence NM_001172566 or in NCBI Reference Sequence NM_002468, specifically, to the nucleotide sequences as set forth in SEQ ID NO:71 or in SEQ ID NO:72 or in SEQ ID NO:73 or in SEQ ID NO:74 or in SEQ ID NO:75, which correspond to the sequences of the above indicated NCBI Reference Sequences of the MYD88 transcript, and also relates to the corresponding amino acid sequence for example as set forth in SEQ ID NO:76 or in SEQ ID NO:77 or in SEQ ID NO:78 or in SEQ ID NO:79 or in SEQ ID NO:80, which correspond to the protein sequences defined in NCBI Protein Accession Reference Sequence NP_001166038 and in NCBI Protein Accession Reference Sequence NP_001166039 and in NCBI Protein Accession Reference Sequence NP_001166040 and in NCBI Protein Accession Reference Sequence NP_001166037 and in NCBI Protein Accession Reference Sequence NP_002459 encoding the MYD88 polypeptide.

The term "MYD88" also comprises nucleotide sequences showing a high degree of homology to MYD88, e.g., nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75 or amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75.

The term "OAS1" refers to the 2'-5'-Oligoadenylate Synthetase 1 gene (Ensembl: ENSG00000089127), for example, to the sequence as defined in NCBI Reference Sequence NM_001320151 or in NCBI Reference Sequence NM_002534 or in NCBI Reference Sequence NM_001032409 or in NCBI Reference Sequence NM_016816, specifically, to the nucleotide sequences as set forth in SEQ ID NO:81 or in SEQ ID NO:82 or in SEQ ID NO:83 or in SEQ ID NO:84, which correspond to the sequences of the above indicated NCBI Reference Sequences of the OAS1 transcript, and also relates to the corresponding amino acid sequences for example as set forth in SEQ ID NO:85 or in SEQ ID NO:86 or in SEQ ID NO:87 or in SEQ ID NO:88, which correspond to the protein sequences defined in NCBI Protein Accession Reference Sequence NP_001307080 and in NCBI Protein Accession Reference Sequence NP_002525 and in NCBI Protein Accession Reference Sequence NP_001027581 and in NCBI Protein Accession Reference Sequence NP_058132 encoding the OAS1 polypeptide.

The term "OAS1" also comprises nucleotide sequences showing a high degree of homology to OAS1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84 or amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84.

The term "PAG1" refers to the Phosphoprotein Membrane Anchor With Glycosphingolipid Microdomains 1 gene (Ensembl: ENSG00000076641), for example, to the sequence as defined in NCBI Reference Sequence NM_018440, specifically, to the nucleotide sequence as set forth in SEQ ID NO:89, which corresponds to the sequence of the above indicated NCBI Reference Sequence of the PAG1 transcript, and also relates to the corresponding amino acid sequence for example as set forth in SEQ ID NO:90, which corresponds to the protein sequence defined in NCBI Protein Accession Reference Sequence NP_060910 encoding the PAG1 polypeptide.

The term "PAG1" also comprises nucleotide sequences showing a high degree of homology to PAG1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:89 or amino acid sequences being at least 75%, 80%, 85%, 90%, 91%, 92%, 93%, 94%, 95%, 96%, 97%, 98% or 99% identical to the sequence as set forth in SEQ ID NO:90 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:90 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:89.

The term "PDE4D" refers to the human Phosphodiesterase 4D gene (Ensembl: ENSG00000113448), for example, to the sequence as defined in NCBI Reference Sequence NM_001104631 or in NCBI Reference Sequence NM_001349242 or in NCBI Reference Sequence NM_001197218 or in NCBI Reference Sequence NM_006203 or in NCBI Reference Sequence NM_001197221 or in NCBI Reference Sequence NM_001197220 or in NCBI Reference Sequence NM_001197223 or in NCBI Reference Sequence NM_001165899 or in NCBI Reference Sequence NM_001165899, specifically, to the nucleotide sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99, which correspond to the sequences of the above indicated NCBI Reference Sequence of the PDE4D transcript, and also relates to the corresponding amino acid sequence for example as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108, which correspond to the protein sequences defined in NCBI Protein Accession Reference Sequence NP_001098101 and in NCBI Protein Accession Reference Sequence NP_001336171 and in NCBI Protein Accession Reference Sequence NP_001184147 and in NCBI Protein Accession Reference Sequence NP_006194 and in NCBI Protein Accession Reference Sequence NP_001184150 and in NCBI Protein Accession Reference Sequence NP_001184149 and in NCBI Protein Accession Reference Sequence NP_001184152 and in NCBI Protein Accession Reference Sequence NP_001159371 and in NCBI Protein Accession Reference Sequence NP_001184148 encoding the PDE4D polypeptide.

The term "PDE4D" also comprises nucleotide sequences showing a high degree of homology to PDE4D, e.g., nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99 or amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99.

The term "PRKACA" refers to the Protein Kinase cAMP-Activated Catalytic Subunit Alpha gene (Ensembl: ENSG00000072062), for example, to the sequence as defined in NCBI Reference Sequence NM_002730 or in NCBI Reference Sequence NM_207518, specifically, to the nucleotide sequences as set forth in SEQ ID NO:109 or in SEQ ID NO: 110, which correspond to the sequences of the above indicated NCBI Reference Sequences of the PRKACA transcript, and also relates to the corresponding amino acid sequence for example as set forth in SEQ ID NO:111 or in SEQ ID NO:112, which correspond to the protein sequences defined in NCBI Protein Accession Reference Sequence NP_002721 and in NCBI Protein Accession Reference Sequence NP_997401 encoding the PRKACA polypeptide.

The term "PRKACA" also comprises nucleotide sequences showing a high degree of homology to PRKACA, e.g., nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110 or amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110.

The term "PRKACB" refers to the Protein Kinase cAMP-Activated Catalytic Subunit Beta gene (Ensembl: ENSG00000142875), for example, to the sequence as defined in NCBI Reference Sequence NM_002731 or in NCBI Reference Sequence NM_182948 or in NCBI Reference Sequence NM_001242860 or in NCBI Reference Sequence NM_001242859 or in NCBI Reference Sequence NM_001242858 or in NCBI Reference Sequence NM_001242862 or in NCBI Reference Sequence NM_001242861 or in NCBI Reference Sequence NM_001300915 or in NCBI Reference Sequence NM_207578 or in NCBI Reference Sequence NM_001242857 or in NCBI Reference Sequence NM_001300917, specifically, to the nucleotide sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123, which correspond to the sequences of the above indicated NCBI Reference Sequences of the PRKACB transcript, and also relates to the corresponding amino acid sequence for example as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134, which correspond to the protein sequences defined in NCBI Protein Accession Reference Sequence NP_002722 and in NCBI Protein Accession Reference Sequence NP_891993 and in NCBI Protein Accession Reference Sequence NP_001229789 and in NCBI Protein Accession Reference Sequence NP_001229788 and in NCBI Protein Accession Reference Sequence NP_001229787 and in NCBI Protein Accession Reference Sequence NP_001229791 and in NCBI Protein Accession Reference Sequence NP_001229790 and in NCBI Protein Accession Reference Sequence NP_001287844 and in NCBI Protein Accession Reference Sequence NP_997461 and in NCBI Protein Accession Reference Sequence NP_001229786 and in NCBI Protein Accession Reference Sequence NP_001287846 encoding the PRKACB polypeptide.

The term "PRKACB" also comprises nucleotide sequences showing a high degree of homology to PRKACB, e.g., nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123 or amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123.

The term "PTPRC" refers to the Protein Tyrosine Phosphatase Receptor Type C gene (Ensembl: ENSG00000081237), for example, to the sequence as defined in NCBI Reference Sequence NM_002838 or in NCBI Reference Sequence NM_080921, specifically, to the nucleotide sequence as set forth in SEQ ID NO:135 or in SEQ ID NO:136, which correspond to the sequences of the above indicated NCBI Reference Sequences of the PTPRC transcript, and also relates to the corresponding amino acid sequence for example as set forth in SEQ ID NO:137 or in SEQ ID NO:138, which correspond to the protein sequences defined in NCBI Protein Accession Reference Sequence NP_002829 encoding the PTPRC polypeptide and in NCBI Protein Accession Reference Sequence NP_563578.

The term "PTPRC" also comprises nucleotide sequences showing a high degree of homology to PTPRC, e.g., nucleic acid sequences being at least 75%, 80%, 85%, 90%, 91%, 92%, 93%, 94%, 95%, 96%, 97%, 98% or 99% identical to the sequence as set forth in SEQ ID NO:135 or in SEQ ID NO:136 or amino acid sequences being at least 75%, 80%, 85%, 90%, 91%, 92%, 93%, 94%, 95%, 96%, 97%, 98% or 99% identical to the sequence as set forth in SEQ ID NO:137 or in SEQ ID NO:138 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:137 or in SEQ ID NO:138 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:135 or in SEQ ID NO:136.

The term "RAP1GAP2" refers to the human RAP1 GTPase Activating Protein 2 gene (ENSG00000132359), for example, to the sequence as defined in NCBI Reference Sequence NM_015085 or in NCBI Reference Sequence NM_001100398 or in NCBI Reference Sequence NM_001330058, specifically, to the nucleotide sequence as set forth in SEQ ID NO:139 or in SEQ ID NO:140 or in SEQ ID NO:141, which correspond to the sequences of the above indicated NCBI Reference Sequences of the RAP1GAP2 transcript, and also relates to the corresponding amino acid sequence for example as set forth in SEQ ID NO:142 or in SEQ ID NO:143 or in SEQ ID NO:144, which correspond to the protein sequences defined in NCBI Protein Accession Reference Sequence NP_055900 and in NCBI Protein Accession Reference Sequence NP_001093868 and in NCBI Protein Accession Reference Sequence NP_001316987 encoding the RAP1GAP2 polypeptide.

The term "RAP1GAP2" also comprises nucleotide sequences showing a high degree of homology to RAP1-GAP2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:139 or in SEQ ID NO:140 or in SEQ ID NO:141 or amino acid sequences being at least 75%, 80%, 85%, 90%, 91%, 92%, 93%, 94%, 95%, 96%, 97%, 98% or 99% identical to the sequence as set forth in SEQ ID NO:142 or in SEQ ID NO:143 or in SEQ ID NO:144 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:142 or in SEQ ID NO:143 or in SEQ ID NO:144 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:139 or in SEQ ID NO:140 or in SEQ ID NO:141.

The term "SLC39A11" refers to the human Solute Carrier Family 39 Member 11 gene (Ensembl: ENSG00000133195), for example, to the sequence as defined in NCBI Reference Sequence NM_139177 or in NCBI Reference Sequence NM_001352692, specifically, to the nucleotide sequence as set forth in SEQ ID NO:145 or in SEQ ID NO:146, which correspond to the sequences of the above indicated NCBI Reference Sequences of the SLC39A11 transcript, and also relates to the corresponding amino acid sequence for example as set forth in SEQ ID NO:147 or in SEQ ID NO:148, which correspond to the protein sequences defined in NCBI Protein Accession Reference Sequence NP_631916 and in NCBI Protein Accession Reference Sequence NP_001339621 encoding the SLC39A11 polypeptide.

The term "SLC39A11" also comprises nucleotide sequences showing a high degree of homology to SLC39A11, e.g., nucleic acid sequences being at least 75%, 80%, 85%, 90%, 91%, 92%, 93%, 94%, 95%, 96%, 97%, 98% or 99% identical to the sequence as set forth in SEQ ID NO:145 or in SEQ ID NO:146 or amino acid sequences being at least 75%, 80%, 85%, 90%, 91%, 92%, 93%, 94%, 95%, 96%, 97%, 98% or 99% identical to the sequence as set forth in SEQ ID NO:147 or in SEQ ID NO:148 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:147 or in SEQ ID NO:148 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:145 or in SEQ ID NO:146.

The term "TDRD1" refers to the human Tudor Domain Containing 1 gene (Ensembl: ENSG00000095627), for example, to the sequence as defined in NCBI Reference Sequence NM_198795, specifically, to the nucleotide sequence as set forth in SEQ ID NO:149, which corresponds to the sequence of the above indicated NCBI Reference Sequence of the TDRD1 transcript, and also relates to the corresponding amino acid sequence for example as set forth in SEQ ID NO:150, which corresponds to the protein sequence defined in NCBI Protein Accession Reference Sequence NP_942090 encoding the TDRD1 polypeptide.

The term "TDRD1" also comprises nucleotide sequences showing a high degree of homology to TDRD1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:149 or amino acid sequences being at least 75%, 80%, 85%, 90%, 91%, 92%, 93%, 94%, 95%, 96%, 97%, 98% or 99% identical to the sequence as set forth in SEQ ID NO:150 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:150 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:149.

The term "TLR8" refers to the Toll Like Receptor 8 gene (Ensembl: ENSG00000101916), for example, to the sequence as defined in NCBI Reference Sequence NM_138636 or in NCBI Reference Sequence NM_016610, specifically, to the nucleotide sequences as set forth in SEQ ID NO:151 or in SEQ ID NO:152, which correspond to the sequences of the above indicated NCBI Reference Sequences of the TLR8 transcript, and also relates to the corresponding amino acid sequences for example as set forth in SEQ ID NO:153 or in SEQ ID NO:154, which corresponds to the protein sequences defined in NCBI Protein Accession Reference Sequence NP_619542 and in NCBI Protein Accession Reference Sequence NP_057694 encoding the TLR8 polypeptide.

The term "TLR8" also comprises nucleotide sequences showing a high degree of homology to TLR8, e.g., nucleic acid sequences being at least 75%, 80%, 85%, 90%, 91%, 92%, 93%, 94%, 95%, 96%, 97%, 98% or 99% identical to the sequence as set forth in SEQ ID NO:151 or in SEQ ID NO:152 or amino acid sequences being at least 75%, 80%, 85%, 90%, 91%, 92%, 93%, 94%, 95%, 96%, 97%, 98% or 99% identical to the sequence as set forth in SEQ ID NO:153 or in SEQ ID NO:154 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:153 or in SEQ ID NO:154 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:151 or in SEQ ID NO:152.

The term "VWA2" refers to the human Von Willebrand Factor A Domain Containing 2 gene (Ensembl: ENSG00000165816), for example, to the sequence as defined in NCBI Reference Sequence NM_001320804, specifically, to the nucleotide sequence as set forth in SEQ ID NO:155, which corresponds to the sequence of the above indicated NCBI Reference Sequence of the VWA2 transcript, and also relates to the corresponding amino acid sequence for example as set forth in SEQ ID NO:156, which corresponds to the protein sequence defined in NCBI Protein Accession Reference Sequence NP_001307733 encoding the VWA2 polypeptide.

The term "VWA2" also comprises nucleotide sequences showing a high degree of homology to VWA2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:155 or amino acid sequences being at least 75%, 80%, 85%, 90%, 91%, 92%, 93%, 94%, 95%, 96%, 97%, 98% or 99% identical to the sequence as set forth in SEQ ID NO:156 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:156 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:155.

The term "ZAP70" refers to the Zeta Chain Of T-Cell Receptor Associated Protein Kinase 70 gene (Ensembl: ENSG00000115085), for example, to the sequence as defined in NCBI Reference Sequence NM_001079 or in NCBI Reference Sequence NM_207519, specifically, to the nucleotide sequences as set forth in SEQ ID NO:157 or in SEQ ID NO:158, which correspond to the sequences of the above indicated NCBI Reference Sequences of the ZAP70 transcript, and also relates to the corresponding amino acid sequence for example as set forth in SEQ ID NO:159 or in SEQ ID NO:160, which corresponds to the protein sequences defined in NCBI Protein Accession Reference Sequence NP_001070 and in NCBI Protein Accession Reference Sequence NP_997402 encoding the ZAP70 polypeptide.

The term "ZAP70" also comprises nucleotide sequences showing a high degree of homology to ZAP70, e.g., nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158or amino acid sequences being at least 75%, 80%, 85%, 90%, 91%, 92%, 93%, 94%, 95%, 96%, 97%, 98% or 99% identical to the sequence as set forth in SEQ ID NO:159 or in SEQ ID NO:160 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:159 or in SEQ ID NO:160 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158.

The term "ZBP1" refers to the Z-DNA Binding Protein 1 gene (Ensembl: ENSG00000124256), for example, to the sequence as defined in NCBI Reference Sequence NM_030776 or in NCBI Reference Sequence NM_001160418 or in NCBI Reference Sequence NM_001160419, specifically, to the nucleotide sequence as set forth in SEQ ID NO:161 or in SEQ ID NO:162 or in SEQ ID NO:163, which corresponds to the sequence of the above indicated NCBI Reference Sequence of the ZBP1 transcript, and also relates to the corresponding amino acid sequence for example as set forth in SEQ ID NO:164 or in SEQ ID NO:165 or in SEQ ID NO:166, which corresponds to the protein sequences defined in NCBI Protein Accession Reference Sequence NP_110403 and in NCBI Protein Accession Reference Sequence NP_001153890 and in NCBI Protein Accession Reference Sequence NP_001153891 encoding the ZBP1 polypeptide.

The term "ZBP1" also comprises nucleotide sequences showing a high degree of homology to ZBP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163 or amino acid sequences being at least 75%, 80%, 85%, 90%, 91%, 92%, 93%, 94%, 95%, 96%, 97%, 98% or 99% identical to the sequence as set forth in SEQ ID NO:164 or in SEQ ID NO:165 or in SEQ ID NO:166 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 164 or in SEQ ID NO: 165 or in SEQ ID NO:166 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 161 or in SEQ ID NO: 162 or in SEQ ID NO: 163.

Each of the IDR_14 genes and TCR_17 genes were tested individually on the dataset, a large portion were found to significantly stratify the patients into groups with a poor predicted outcome e.g. an overall predicted survival time of less than 30 months with a probability of 50%, or a good predicted outcome, e.g. an overall predicted survival time of more than 40 months with a probability of 50%. For the individual genes a cut-off P-value of 0.05 was used to indicate a significant predictor. The significant genes and their P-values are listed below:

| gene | p value |
| --- | --- |
| CD4 | 2.4E-09 |
| IFIT3 | 0.000006 |
| EZR | 0.000007 |
| ZAP70 | 0.0000075 |
| IFIH1 | 0.000032 |
| LCK | 0.000067 |

(continued)

| gene | p value |
| --- | --- |
| CD3G | 0.00011 |
| CD28 | 0.00011 |
| APOBEC3A | 0.00025 |
| OAS1 | 0.00029 |
| CD247 | 0.00052 |
| CD2 | 0.00056 |
| IFI16 | 0.0006 |
| PAG1 | 0.00077 |
| PTPRC | 0.00081 |
| ZBP1 | 0.00086 |
| CD3E | 0.0011 |
| LRRFIP1 | 0.0015 |
| TLR8 | 0.0021 |
| DDX58 | 0.0029 |
| DHX9 | 0.0033 |
| AIM2 | 0.0036 |
| FYN | 0.0098 |
| IFIT1 | 0.014 |

Therefore, in an alternative aspect the invention relates to a method of predicting an optimal treatment strategy for a subject having melanoma, the method comprising: determining or receiving the result of a determination of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and determining the prediction of the outcome based on one or more gene expression levels, wherein the outcome is a poor outcome or a good outcome. Such predicted outcome may be useful in deciding a treatment strategy for the patient. For example, for a poor outcome a more aggressive treatment may be recommended, while for a good predicted outcome standard of care treatment (e.g. radiotherapy, surgery, chemotherapy, targeted therapy, immune checkpoint inhibitor therapy, or combinations thereof) may be recommended. In a particularly preferred embodiment the recommended treatment in case of a poor predicted outcome comprises administering the ICI Pembroluzimab or an equivalent thereof, however it is understood that this treatment may be combined with other treatments such as but not limited to radiotherapy, surgery, chemotherapy, targeted therapy, combination immunotherapy, or combinations of the aforementioned options.

In an embodiment the predicted outcome is a good outcome the suggested treatment strategy comprises administering or performing one or more of the therapies selected from immune checkpoint inhibitor based therapy, chemotherapy, targeted therapy, surgery or radiation therapy.

In an embodiment the one or more for example 1, 2, 3, 4, 5, 6, 7, 8, 9, 10, 11, 12, gene expression levels are selected from CD4, IFIT3, EZR, ZAP70, IFIH1, LCK, CD3G, CD28, APOBEC3A, OAS1, CD247, CD2, IFI16, PAG1, PTPRC, and ZBP1, preferably selected from CD4, IFIT3, EZR, ZAP70, IFIH1, and LCK. In an embodiment the one or more, for example 1, 2, 3, 4, 5, 6, 7, 8, 9, 10, 11, 12, or more gene expression levels further comprises one or more, for example 1, 2, 3, 4, 5, 6, 7 or all 7, gene expression levels selected from CIAO1, MYD88, CSK, LAT, PDE4D, PRKACA and PRKACB. In an embodiment the prediction is based on six or more, for example 6, 7, 8, 9, 10, 11, 12, or more, gene expression levels selected from AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, and ZAP70.

As indicated above the examples and data included in the application make it plausible that any combination of six genes selected from the previously described IDR14 and TCR17 gene signatures provided herein can be used to predict an outcome for a subject having melanoma. Thus in an embodiment the invention describes a method of predicting an outcome of a subject having melanoma, the method comprising: determining or receiving the result of a determination of six or more gene expression levels selected from AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, and ZAP70; said gene expression levels being determined in a biological sample obtained from the subject; and determining the prediction of the outcome based on six or more gene expression levels. In an embodiment the outcome is determining a treatment response to an immune checkpoint inhibitor, preferably Pembroluzimab. The inventors analyzed the predictions models and identified genes that contribute most in each of the IDR14 and TCR17 models. Although inclusion of at least one preferred gene is not needed for accurate prediction it is hypothesized that doing so may increase accuracy of the model. Therefore, in an embodiment the six or more gene expression levels comprise at least one, preferably two, three, four, five or six gene expression level(s) selected from: CD4, IFIT3, EZR, ZAP70, IFIH1, LCK, CD3G, CD28, APOBEC3A, OAS1, CD247, CD2, IFI16, PAG1, PTPRC, and ZBP1, preferably selected from CD4, IFIT3, EZR, ZAP70, IFIH1, and LCK,

In an embodiment the six or more gene expression levels comprise at least one, preferably two, three, four, five or six gene expression level(s) selected from:

AIM2, CIAO1, DHX9, IFI16, LRRFIP1 and OAS1 (IDR14_6.1 model genes); or
APOBEC3A, DDX58, IFIT1, MYD88, TLR8 and ZBP1 (IDR14_6.2 model genes); or
DHX9, IFI16, IFIH1, IFIT3, MYD88 and TLR8 (IDR14_6.3 model genes); or
CD247, CD3E, EZR, LAT, PDE4D and PRKACA (TCR17_6.1 model genes); or
CD28, CD4, FYN, PAG1, PRKACB and ZAP70 (TCR17_6.2 model genes); or
CD2, CD3G, CSK, EZR, LCK and PTPRC (TCR17_6.3 model genes); or
AIM2, IFIH1, TLR8, CD3E, EZR, and PDE4D (SKCMAI_6.1 model genes); or
CIAO1, DHX9, OAS1, CD247, CSK and FYN (SKCMAI_6.2 model genes); or
DDX58, IFIT3, MYD88, CD28, CD4 and ZAP70 (SKCMAI_6.3 model genes).

In an embodiment the determining of the prediction of the outcome further comprises combining the six or more gene expression levels with a regression function that had been derived from a population of subjects having melanoma. In an embodiment the determining of the outcome is further based on one or more clinical parameters obtained from the subject.

Thus in a further embodiment the invention relates to a method of predicting an optimal treatment strategy for a subject having melanoma, the method comprising: determining or receiving the result of a determination of six or more gene expression levels selected from AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, and ZAP70; said gene expression levels being determined in a biological sample obtained from the subject; and determining the prediction of the outcome based on the six or more gene expression levels, wherein the outcome is a poor outcome or a good outcome. Preferably when the predicted outcome is a poor outcome the suggested treatment strategy comprises administering Pembroluzimab or an equivalent thereof.

In an embodiment the method further comprises a step of providing the prediction to a medical caregiver or the subject.

In an embodiment the biological sample(s) is/are obtained from the subject before the start of a therapy. In an embodiment the biological sample is a blood sample or a biopsy, preferably a biopsy from the tumor or a metastasis thereof, obtained from the subject.

In a third aspect the invention relates to apparatus for predicting an outcome for a subject having a melanoma, comprising: an input adapted to receive data indicative of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and a processor adapted to determine the prediction of the outcome based on the one or more gene expression levels, wherein the outcome is a poor outcome or a good outcome. In an embodiment the processor is further adapted to provide a treatment recommendation, wherein the treatment recommendation comprises providing Pembroluzimab or an equivalent thereof to the subject when the predicted outcome is a poor outcome.

In a fourth aspect the invention relates to a computer program product comprising instructions which, when the program is executed by a computer, cause the computer to carry out a method for predicting an outcome for a subject having a melanoma comprising: receiving data indicative of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and determining a prediction of the outcome based on the one or more gene expression levels wherein the outcome is a poor outcome or a good outcome. In an embodiment the method further comprises providing a treatment recommendation to the subject, wherein when the predicted outcome is a poor outcome the treatment recommendation comprises administering Pembroluzimab or an equivalent thereof.

In a fifth aspect the invention relates to the use of a kit for predicting an outcome for a subject having a melanoma, the use comprising: determining one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1 in a sample obtained from a subject having a melanoma; and predicting the outcome based on the one or more gene expression levels wherein the outcome is a poor outcome or a good outcome, wherein the kit comprises means for determining one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1. In an embodiment the use further comprises providing a treatment recommendation, wherein if the predicted outcome is a poor outcome the treatment recommendation comprises administering Pembroluzimab or an equivalent thereof.

The means for determining the expression levels of the target genes may be primers and/or probes suitable for any one of PCR, quantitative PCR, digital PCR, RNA sequencing, or targeted RNA sequencing. Thus for example the means may be PCR primers, quantitative PCR primers and probes, digital PCR primers and probes, or capture probes for targeted mRNA sequencing. Preferably the means are PCR amplification primers and optionally probes or mRNA targeted sequencing capture probes.

Alternatively, the invention relates to a method of treating, preventing or ameliorating melanoma, the method comprising determining an outcome for the subject having melanoma using the method as defined in the second aspect of the invention, and administering to the subject the therapy based on the outcome; wherein the therapy is selected from a standard of care treatment when the predicted outcome is favorable, or wherein the therapy is Pembroluzimab or an equivalent thereof when the predicted outcome is unfavorable.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t) = H_0(t) \cdot exp(w_1 \cdot V_1 + w_2 \cdot V_2 + w_3 \cdot V_3 + ...)$, where $V_i$, $V_2$, $V_3$ ... are predictor variables and $H_0(t)$ is the baseline hazard while $H(t)$ is the hazard at any time t. The hazard ratio (or the risk to reach the event) is represented by $Ln[H(t)/ H_0(t)] = w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

In one particular realization, the combination of the first gene expression profiles for the six or more, for example 6, 7, 8, 9, 10, 11, 12, 13 or all, of the immune defense response genes with a regression function is determined as follows:
IDR_model:

$$(w_1 \cdot AIM2) + (w_2 \cdot APOBEC3A) + (w_3 \cdot CIAO1) + (w_4 \cdot DDX58) + (w_5 \cdot DHX9) + (w_6 \cdot IFI16) + (w_7 \cdot IFIH1) + (w_8 \cdot IFIT1) + (w_9 \cdot IFIT3) \ (1) + (w_{10} \cdot LRRFIP1) + (w_{11} \cdot MYD88) + (w_{12} \cdot OAS1) + (w_{13} \cdot TLR8) + (w_{14} \cdot ZBP1) \tag{1}$$

where $w_1$ to $w_{14}$ are weights and AIM2, APOBEC3A, CIAO1, DDX58, DHX9, IFI16, IFIH1, IFIT1, IFIT3, LRRFIP1, MYD88, OAS1, TLR8, and ZBP1 are the expression levels of the immune defense response genes.

In one particular realization, the combination of the second gene expression profiles for the six or more, for example, 6, 7, 8, 9, 10, 11, 12, 13, 14, 15, 16 or all, of the T-Cell receptor signaling genes with a regression function is determined as follows:
TCR_SIGNALING_model:

$$(w_{15} \cdot C2) + (w_{16} \cdot CD247) + (w_{17} \cdot CD28) + (w_{is} \cdot CD3E) + (w_{19} \cdot CD3G) + (w_{20} \cdot CD4) + (w_{21} \cdot CSK) + (w_{22} \cdot EZR) + (w_{23} \cdot FYN) + (2) \ (w_{24} \cdot LAT) + (w_{25} \cdot LCK) + (w_{26} \cdot PAG1) + (w_{27} \cdot PDE4D) + (w_{28} \cdot PRKACA) + (w_{29} \cdot PRKACB) + (w_{30} \cdot PTPRC) + (w_{31} \cdot ZAP70) \tag{2}$$

where $w_{15}$ to $w_{31}$ are weights and CD2, CD247, CD28, CD3E, CD3G, CD4, CSK, EZR, FYN, LAT, LCK, PAG1, PDE4D, PRKACA, PRKACB, PTPRC, and ZAP70 are the expression levels of the T-Cell receptor signaling genes.

It is further preferred that the determining of the prediction of the outcome further comprises combining the combination of the first gene expression profiles and the combination of the second gene expression profiles with a regression function that had been derived from a population of melanoma subjects.

In one particular realization, the prediction of the outcome is determined as follows:

$$SKCMAI\_model:$$
$$(w_{32} \cdot IDR\_model) + (w_{33} \cdot TCR\_SIGNALING\_model) \tag{3}$$

where $w_{32}$ to $w_{33}$ are weights, IDR_model is the above-described regression model based on the expression profiles for the two or more, for example, 2, 3, 4, 6, 7, 8, 9, 10, 11, 12, 13 or all, of the immune defense response genes, and TCR_SIGNALING_model is the above-described regression model based on the expression profiles for the two or more, for example, 2, 3, 4, 6, 7, 8, 9, 10, 11, 12, 13, 14, 15, 16 or all, of the T-Cell receptor signaling genes.

In one particular realization, the combination of the expression levels of the six or more genes are calculated as follows:
SKCMAI_6 model:

$$(w_a \cdot [geneA]) + (w_b \cdot [geneB]) + (w_c \cdot [geneC]) + (w_d \cdot [geneD]) + (w_e \cdot [geneE]) + (w_f \cdot [geneF])$$

where $w_x$ represents the weight for the respective gene geneX. It is within the ability of the skilled artisan to generate combinations of six or more genes as defined herein and calculated the respective weights for such model.

The prediction of the outcome may also be classified or categorized into one of at least two risk groups, based on the value of the prediction of the outcome. For example, there may be two risk groups, or three risk groups, or four risk groups, or more than four predefined risk groups. Each risk group covers a respective range of (non-overlapping) values of the prediction of the outcome. For example, a risk group may indicate a probability of occurrence of a specific clinical event from 0 to <0.1 or from 0.1 to <0.25 or from 0.25 to <0.5 or from 0.5 to 1.0 or the like.

It is further preferred that the determining of the prediction of the outcome is further based on one or more clinical parameters obtained from the subject.

As mentioned above, various measures based on clinical parameters have been investigated. By further basing the prediction of the outcome on such clinical parameter(s), it can be possible to further improve the prediction.

It is preferred that the biological sample is obtained from the subject before the start of the therapy. The gene expression profile(s) may be determined in the form of mRNA or protein in tissue of the melanoma. Alternatively, if the genes are present in a soluble form, the gene expression profile(s) may be determined in blood, for example a blood sample comprising a circulating tumor cell.

It shall be understood that the method of predicting an outcome of a subject having a melanoma as broadly described herein, the apparatus as broadly described herein, the computer program product as broadly described herein, the diagnostic kit as broadly described herein, the use of the diagnostic kit as broadly described herein, the use of one or more gene expression levels as broadly described herein and the product for use as broadly described herein have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

Below is described schematically and exemplarily a workflow of an embodiment of a method of predicting an outcome of a subject having a melanoma.

A biological sample is obtained from each of a first set of patients (subjects) diagnosed with a melanoma. Preferably, monitoring melanoma has been performed for these patients over a period of time, such as at least one year, or at least two years, or about five years, after obtaining the biological sample.

In a next step, a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two or more genes which can be normalized using value(s) for each of a set of reference genes, such as B2M, HPRT1, POLR2A, and/or PUM1. In one realization, the gene expression level for each of the two or more genes of the first gene expression profiles, and/or the second gene expression profiles, and/or the third gene expression profiles is normalized with respect to one or more reference genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these reference genes.

In a next step, a regression function for assigning a prediction of the outcome is determined based on the first gene expression profiles for the two or more immune defense response genes, AIM2, APOBEC3A, CIAO1, DDX58, DHX9, IFI16, IFIH1, IFIT1, IFIT3, LRRFIP1, MYD88, OAS1, TLR8, and/or ZBP1, and/or the second gene expression profiles for the two or more T-Cell receptor signaling genes, CD2, CD247, CD28, CD3E, CD3G, CD4, CSK, EZR, FYN, LAT, LCK, PAG1, PDE4D, PRKACA, PRKACB, PTPRC, and/or ZAP70, obtained for at least some of the biological samples obtained for the first set of patients and respective results obtained from the monitoring. In one particular realization, the regression function is determined as specified above.

In a next step, a biological sample is provided from a patient (subject or individual).

In a next step, a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, is determined, e.g., by performing PCR on the biological sample. In one realization, the gene expression level for each of the two or more genes of the first gene expression profiles, and/or the second gene expression profiles, and/or the third gene expression profiles is normalized with respect to one or more reference genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these reference genes.

In a next step, a prediction of the outcome based on the first and second gene expression profiles is determined for the patient using the regression function.

In a next step, a therapy recommendation may be provided, e.g., to the patient or his or her guardian, to a doctor, or to another healthcare worker, based on the prediction or the personalization. To this end, the prediction or personalization may be categorized into one of a predefined set of risk groups, based on the value of the prediction or personalization.

In one embodiment, the gene expression profiles are determined by detecting mRNA expression using two or more primers and/or probes and/or two or more sets thereof.

Based on the data provided in the Examples and Figures it is shown that the identified molecules will individually provide predictive value with regard to the effectiveness of the treatment of melanomas. Therefore in an embodiment the method is based on for example one or more gene expression levels, such as preferably one, two , three, four, five, six, seven eight nine or ten expression levels selected from the immune defense response genes AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signaling genes selected from CD2, CD247, CD28, CD3E, CD3G, CD4, CSK, EZR, FYN, LAT, LCK, PAG1, PDE4D, PRKACA, PRKACB, PTPRC, and ZAP70.

EXAMPLES

### Example 1 - Dataset

The following dataset has been used to calibrate the Cox model weights:

**TGCA Cohort;** This cohort comprises data from patients having skin cutaneous melanoma. The number of patients having Tumor T stages: #8 T.IS; #23 T0; #42 T1; #78 T2; #90 T3; #153 T4; #76 NA, the number of patients having Tumor N stages: #235 N0; #74 N1; #49 N2; #55 N3; #57 NA, the number of patients having Tumor M stages: #418 M0; #24 M1; #28 NA. 180 patients are female, 290 are male. The median follow-up in the cohort is 39 months; with a max follow-up of 374 months. 89.4% of patients did not receive radiation therapy, 10.4% did, for 0.2% it is unknown.

### Example 2 - Individual gene expression levels

It was evaluated if individual genes form the IDR_14 or TCR_17 genes signatures are significantly associated with survival. The results are depicted in Figs. 1- 31 and show Kaplan-Meier curves based on single gene expression (high or low) in a 198 patient cohort with all patients having a melanoma. The clinical endpoint tested was overall survival time in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in a train set, and were fixed for validation in the test cohort (shown here). Logrank, HR and confidence interval are included in the figure. The included supplementary lists indicate the number of patients at risk. Panels 1-31 respectively show prediction for the following genes (also indicated in the respective graphs): AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, ZAP70.

The following genes were found significantly associated with survival (P < 0.05):

| gene | p value |
| --- | --- |
| CD4 | 2.4E-09 |
| IFIT3 | 0.000006 |
| EZR | 0.000007 |
| ZAP70 | 0.0000075 |
| IFIH1 | 0.000032 |
| LCK | 0.000067 |

(continued)

| gene | p value |
| --- | --- |
| CD3G | 0.00011 |
| CD28 | 0.00011 |
| APOBEC3A | 0.00025 |
| OAS1 | 0.00029 |
| CD247 | 0.00052 |
| CD2 | 0.00056 |
| IFI16 | 0.0006 |
| PAG1 | 0.00077 |
| PTPRC | 0.00081 |
| ZBP1 | 0.00086 |
| CD3E | 0.0011 |
| LRRFIP1 | 0.0015 |
| TLR8 | 0.0021 |
| DDX58 | 0.0029 |
| DHX9 | 0.0033 |
| AIM2 | 0.0036 |
| FYN | 0.0098 |
| IFIT1 | 0.014 |

**Example 3** - **Construction of the SKCMAI model**

_Generation and validation of gene signature based models_

For each gene, the log2 expression value as provided in the download from the TCGA database (TCGA Melanoma) was obtained.

The log2 expression values for each gene were transformed into z-scores by calculating:

$$\log2\_gene \text{ transformed z-score} = ((\log2\_gene) - (mean\_samples))/(stdev\_samples) \tag{6}$$

where log2_gene is the log2 gene expression value per gene, mean_samples is the mathematical mean of the log2_gene values across all samples, and stdev_samples is the standard deviation of the log2_gene values across all samples.

This process distributes the transformed log2_gene values around the mean 0 with a standard deviation of 1.

For the multivariate analysis of the genes of interest we used the log2_gene transformed z-score value of each gene as input.

_Cox Regression Analysis_

We then set out to test whether the combination of the 14 immune defense response genes and the 17 T-Cell receptor signaling genes will exhibit a prognostic value for melanoma. With Cox regression we modelled the expression levels of the 14 immune defense response genes, and of the 17 T-Cell receptor signaling genes respectively, to overall survival in a cohort of 93 melanoma cancer patients.

The Cox regression functions were derived as follows: IDR_model:

$(w_1 \cdot AIM2) + (w_2 \cdot APOBEC3A) + (w_3 \cdot CIAO1) + (w_4 \cdot DDX58) + (w_5 \cdot DHX9) + (w_6 \cdot IFI16) + (w_7 \cdot IFIH1) + (w_8 \cdot IFIT1) + (w_9 \cdot IFIT3) + (w_{10} \cdot LRRFIP1) + (w_{11} \cdot MYD88) + (w_{12} \cdot OAS1) + (w_{13} \cdot TLR8) + (w_{14} \cdot ZBP1)$

TCR_SIGNALING_model:

$(w_{15} \cdot C2) + (w_{16} \cdot CD247) + (w_{17} \cdot CD28) + (w_{18} \cdot CD3E) + (w_{19} \cdot CD3G) + (w_{20} \cdot CD4) + (w_{21} \cdot CSK) + (w_{22} \cdot EZR) + (w_{23} \cdot FYN) + (w_{24} \cdot LAT) + (w_{25} \cdot LCK) + (w_{26} \cdot PAG1) + (w_{27} \cdot PDE4D) + (w_{28} \cdot PRKACA) + (w_{29} \cdot PRKACB) + (w_{30} \cdot PTPRC) + (w_{31} \cdot ZAP70)$

The details for the weights $w_1$ to $w_{31}$ are shown in the following TABLE 4.

TABLE 4: Variables and weights for the three individual Cox regression models as trained on the TGCA Melanoma dataset, i.e., the immune defense response model (IDR_model), the T-Cell receptor signaling model (TCR SIGNALING model) for melanoma; NA - not available.

| Model | | IDR_model | TCR_ SIGNALING_ model |
|---|---|---|---|
| AIM2 | $w_1$ | 0.051 | NA |
| APOBEC3A | $w_2$ | 0.042 | NA |
| CIAO1 | $w_3$ | -0.13 | NA |
| DDX58 | $w_4$ | -0.096 | NA |
| DHX9 | $w_5$ | -0.095 | NA |
| IFI16 | $w_6$ | 0.31 | NA |
| IFIH1 | $w_7$ | 0.43 | NA |
| IFIT1 | $w_8$ | -0.30 | NA |
| IFIT3 | $w_9$ | -0.60 | NA |
| LRRFIP1 | $w_{10}$ | -0.098 | NA |
| MYD88 | $w_{11}$ | 0.53 | NA |
| OAS1 | $w_{12}$ | 0.80 | NA |
| TLR8 | $w_{13}$ | -0.041 | NA |
| ZBP1 | $w_{14}$ | -0.19 | NA |
| CD2 | $w_{15}$ | NA | 0.15 |
| CD247 | $w_{16}$ | NA | 0.11 |
| CD28 | $w_{17}$ | NA | 0.62 |
| CD3E | $w_{18}$ | NA | -0.50 |
| CD3G | $w_{19}$ | NA | -0.11 |
| CD4 | $w_{20}$ | NA | 0.18 |
| CSK | $w_{21}$ | NA | -0.30 |
| EZR | $w_{22}$ | NA | -0.12 |
| FYN | $w_{23}$ | NA | 0.059 |
| LAT | $w_{24}$ | NA | -0.14 |
| LCK | $w_{25}$ | NA | -0.31 |
| PAG1 | $w_{26}$ | NA | -0.24 |
| PDE4D | $w_{27}$ | NA | 0.31 |
| PRKACA | $w_{28}$ | NA | 0.23 |
| PRKACB | $w_{29}$ | NA | -0.27 |
| PTPRC | $w_{30}$ | NA | 0.42 |
| ZAP70 | $w_{31}$ | NA | 0.53 |

Based on the two individual Cox regression models (IDR_model, TCR_SIGNALING_model) we then again used Cox regression to model the combination thereof to overall survival (SKCMAI_model) in the respective cohorts of melanoma patients. We tested the two models in Kaplan-Meier survival analysis.

The Cox regression functions were derived as follows: SKCMAI_model:

$$(w_{32} \cdot IDR\_model) + (w_{33} \cdot TCR\_SIGNALING\_model)$$

The details for the weights $w_{32}$ to $w_{33}$ are shown in the following TABLE 5.

TABLE 5: Variables and weights for two combination Cox regression models, i.e., melanoma AI model (SKCMAI_model); NA - not available.

| Variable | Weights | |
|---|---|---|
| **Model** | | **SKCMAI_model** |
| **IDR_model** | $W_{32}$ | 0.39 |
| **TCR_SIGNALING_model** | $W_{33}$ | 0.54 |

**Example 4** - **Kaplan-Meier Survival Analysis**

For Kaplan-Meier survival curve analysis, the Cox functions of the risk models (IDR_14 model, TCR_17 model, SKCMAI model, 6 gene models, single gene models) were categorized into two sub-cohorts based on a cut-off. The threshold for group separation into low and high risk was based on the risk to experience the clinical endpoint (outcome) as predicted by the respective Cox regression model

The Kaplan-Meier survival curve analysis as shown in Figs. 1 to 46 demonstrate the presence of different patient risk groups. The risk group of a patient is determined by the probability to suffer from the respective clinical endpoint (overall survival) as calculated by the respective risk model as shown in the figures. Depending on the predicted risk of a patient (i.e., depending on in which risk group the patient may belong) to die from melanoma different types of interventions might be indicated. In the low risk group (probability <0.5) standard of care (SOC) delivers acceptable long-term oncological control. This is definitely not the case for the patient group with a risk >0.5 to experience any of the relevant outcomes. In this patient group treatment with Pembroluzimab (or an equivalent thereof) has been shown herein to greatly increase survival time. In addition, escalation of intervention or application of alternative treatment may be provided. Alternative options for treatment escalation are adjuvant therapies with radiation or cytotoxic drugs.

*Discussion*

The effectiveness of therapies for melanoma is limited, resulting in disease progression and ultimately death of patients, especially for those at high risk of recurrence of disease after primary intervention. The prediction of the therapy outcome is very challenging as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the treatment response in order to increase the success rate of melanoma therapies.

We have identified molecules of which expression shows a significant relation to mortality, as well as mortality of treatment response to an immune checkpoint inhibitor of melanomas and therefore are expected to improve the prediction of the effectiveness of such treatments. This can be achieved by guiding patients with lower or high risk of progressive disease and subsequent death from cancer to the most appropriate, potentially more effective form of treatment as compared to currently applied standard of care. This would reduce suffering for those patients who would be spared ineffective therapy, improve chances for patients requiring more aggressive treatment, and would reduce cost spent on ineffective therapies.

Figs. 36 and 37 shows a Kaplan-Meier survival curves for a 367 patient training cohort (Fig. 36) and a 91 patient test cohort (Fig. 37) based on low or high SKCMAI score. A low score (cut-off 0) significantly corresponds to a good outcome while a high score indicates a poor outcome.

**Example 5-6 gene models.**

Next it was hypothesized that a smaller subset of genes would still have predictive power. It is expected that a model of six genes selected from the Immune defense response signature, and/or the T-cell signaling signature as described herein, suffices to make a prediction.

In order to substantiate this random models of six genes selected from either the separate gene signatures (IDR14, TCR17) or from each of the gene signatures as described below. Three randomly selected 6 gene models were generated per gene signature and for the combination set, that were deemed representative for the whole signature and at least covered each gene in the signatures at least once.

Predictive models were generated by assigning weights to each gene in the 6 gene signatures as indicated below.

| Model | Gene | Weight |
|---|---|---|
| IDR14_6.1 | AIM2 | -0.06071 |
| | CIAO1 | 0.125 |
| | DHX9 | 0.06077 |
| | IFI16 | 0.1477 |
| | LRRFIP1 | -0.2246 |
| | OAS1 | 0.00007194 |
| IDR14_6.2 | APOBEC3A | -0.04937 |
| | DDX58 | 0.08368 |
| | IFIT1 | -0.03985 |
| | MYD88 | -0.1104 |
| | TLR8 | -0.007197 |
| | ZBP1 | -0.1908 |
| IDR14_6.3 | DHX9 | -0.009995 |
| | IFI16 | 0.0872 |
| | IFIH1 | 0.01718 |
| | IFIT3 | -0.06421 |
| | MYD88 | -0.1774 |
| | TLR8 | -0.07612 |
| TCR17_6.1 | CD247 | 0.03659 |
| | CD3E | -0.227 |
| | EZR | -0.09092 |
| | LAT | 0.03879 |
| | PDE4D | 0.01681 |
| | PRKACA | 0.2406 |
| TCR17_6.2 | CD28 | -0.08797 |
| | CD4 | -0.002109 |
| | FYN | -0.09005 |
| | PAG1 | 0.05424 |
| | PRKACB | -0.04338 |
| | ZAP70 | -0.1701 |
| TCR17_6.3 | CD2 | 0.1072 |
| | CD3G | -0.311 |
| | CSK | -0.02169 |
| | EZR | -0.09874 |
| | LCK | -0.09442 |
| | PTPRC | 0.08397 |
| SKCMAI _6.1 | AIM2 | 0.01517 |
| | IFIH1 | -0.1274 |
| | TLR8 | 0.01721 |
| | CD3E | -0.2026 |
| | EZR | -0.07275 |
| | PDE4D | -0.0002248 |
| SKCMAI _6.2 | CIAO1 | 0.1527 |
| | DHX9 | 0.03293 |
| | OAS1 | -0.1082 |
| | CD247 | -0.09584 |
| | CSK | -0.171 |
| | FYN | -0.1513 |

36

(continued)

| Model | Gene | Weight |
|---|---|---|
| SKCMAI_6.3 | DDX58 | 0.08128 |
| | IFIT3 | -0.07699 |
| | MYD88 | -0.114 |
| | CD28 | -0.08551 |
| | CD4 | 0.0582 |
| | ZAP70 | -0.1784 |

Each of the 6 gene models could significantly distinguish patient populations with a poor or a good outcome. The data are described in Figs. 38-46 and show that each of the randomly selected 6 genes result in a significant model with p value below 0.001.

**Example 6** - **Predicting outcome of specific ICI treatments**

Next the inventors reviewed whether differences can be observed in patient populations receiving either Pembrolizumab or Nivolumab as a treatment. To do so the patient cohort was split in patients that received Pembrolizumab or Nivolumab, and each sub-cohort was subsequently analyzed using the SKCMAI model for survival. The results are depicted in Figs. 47 and 48. Next the patient cohort was divided based on the SKCMAI risk score and subsequently plotted based on received ICI treatment. These data are depicted in Figs. 49 and 50. A combination of the graphs is included in Figs. 51 and 52. From these data it can clearly be observed that for patients who have a good prospect (low SKCMAI score), there is no difference which immunotherapy (Pembrolizumab or Nivolumab) they received, as the outcome does not significantly deviate. However, for those patients who have a high SKCMAI score (poor predicted outcome), those receiving Pembrolizumab have a significantly better outlook and mean survival time compared to those receiving Nivolumab. Thus it can be concluded that for patients with a poor predicted outcome (e.g. a high SKCMAI score) the preferred treatment Pembrolizumab.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 2 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the methods described herein, can be used to implement one or more steps of the method of risk stratification for therapy selection in a patient with melanoma as illustrated. As will be appreciated, while the steps of the disclosed methods may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

**The attached Sequence Listing, entitled 2023PF00803_SEQ_LST_ST26 is incorporated herein by reference, in its entirety.**

**Claims**

1. Pembroluzimab or an equivalent thereof for use in the treatment of a melanoma in a subject, the use comprising:

   determining or receiving the result of a determination of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1;
   said gene expression levels being determined in a biological sample obtained from the subject; and
   determining the prediction of the outcome based on one or more gene expression levels, wherein the outcome is a poor outcome or a good outcome, and
   when the predicted outcome is a poor outcome Pembroluzimab or an equivalent thereof is administered to the subject.

2. Pembroluzimab or an equivalent thereof for use according to claim 1, wherein the prediction is based on six or more gene expression levels selected from AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, ZAP70.

3. Pembroluzimab or an equivalent thereof for use according to claim 1 or 2, wherein the biological sample is obtained from the subject before the start of a therapy, preferably wherein the biological sample is a blood sample or a biopsy, preferably a biopsy from the tumor or a metastasis thereof, obtained from the subject.

4. Pembroluzimab or an equivalent thereof for use according to any one of claims 1 to 3, wherein the Pembroluzimab or equivalent thereof is combined with Ipilimumab or an equivalent thereof.

5. A method of predicting an outcome for a subject having a melanoma, the method comprising:

   - determining or receiving the result of a determination of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1; said gene expression levels being determined in a biological sample obtained from the subject; and
   - determining the prediction of the outcome based on one or more gene expression levels, wherein the outcome is a poor outcome or a good outcome.

6. The method according to claim 5 wherein the outcome is used to recommend a treatment strategy, wherein if the predicted outcome is a poor outcome the suggested treatment strategy comprises administering Pembroluzimab or an equivalent thereof

7. The method according to claim 6, wherein if the predicted outcome is a good outcome the suggested treatment strategy comprises administering or performing one or more of the therapies selected from immune checkpoint inhibitor based therapy, chemotherapy, targeted therapy, surgery or radiation therapy.

8. The method according to claim 5 or 6, wherein the one or more gene expression levels are selected from CD4, IFIT3, EZR, ZAP70, IFIH1, LCK, CD3G, CD28, APOBEC3A, OAS1, CD247, CD2, IFI16, PAG1, PTPRC, and ZBP1, preferably selected from CD4, IFIT3, EZR, ZAP70, IFIH1, and LCK.

9. The method according to any one of claims 5 to 8, wherein the prediction is based on six or more gene expression levels selected from AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, ZAP70.

10. The method according to any one of claims 5 to 9, further comprising the step of providing the prediction to a medical caregiver or the subject.

11. The method according to any one of claims 5 to 10, wherein the biological sample(s) is/are obtained from the subject before the start of a therapy.

12. The method according to any one of claims 5 to 11, wherein the biological sample is a blood sample or a biopsy, preferably a biopsy from the tumor or a metastasis thereof, obtained from the subject.

13. An apparatus for predicting an outcome for a subject having a melanoma, comprising:

- an input adapted to receive data indicative of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1;
said gene expression levels being determined in a biological sample obtained from the subject; and
- a processor adapted to determine the prediction of the outcome based on the one or more gene expression levels, wherein the outcome is a poor outcome or a good outcome.

14. A computer program product comprising instructions which, when the program is executed by a computer, cause the computer to carry out a method for predicting an outcome for a subject having a melanoma comprising:

- receiving data indicative of one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1;
said gene expression levels being determined in a biological sample obtained from the subject; and
- determining a prediction of the outcome based on the one or more gene expression levels wherein the outcome is a poor outcome or a good outcome.

15. Use of a kit for predicting an outcome for a subject having a melanoma, the use comprising:

- determining one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1 in a sample obtained from a subject having a melanoma; and
- predicting the outcome based on the one or more gene expression levels wherein the outcome is a poor outcome or a good outcome,
wherein the kit comprises means for determining one or more gene expression levels selected from CD4, IFIT3, EZR, ZAP70, IFIH1, LCK, CD3G, CD28, APOBEC3A, OAS1, CD247, CD2, IFI16, PAG1, PTPRC, ZBP1, CD3E, LRRFIP1, TLR8, DDX58, DHX9, AIM2, FYN, and IFIT1.

**Fig. 1**

AIM2

**Fig. 2**

APOBEC3A

**Fig. 3**

CIAO1

HR = 1.68 (0.93 - 3.02)
logrank P = 0.081

Probability

Overall survival time (months)

Expression
low
high

Number at risk
| | | | | | | |
|---|---|---|---|---|---|---|
| low | 41 | 32 | 28 | 10 | 3 | 3 |
| high | 125 | 90 | 56 | 20 | 6 | 3 |

**Fig. 4**

DDX58

HR = 0.39 (0.2 - 0.74)
logrank P = 0.0029

Probability

Overall survival time (months)

Expression
low
high

Number at risk
| | | | | | | |
|---|---|---|---|---|---|---|
| low | 123 | 86 | 55 | 18 | 5 | 2 |
| high | 43 | 36 | 29 | 12 | 4 | 4 |

**Fig. 5**

DHX9

HR = 0.5 (0.31 - 0.8)
logrank P = 0.0033

Expression
low
high

Probability

Overall survival time (months)

Number at risk
| | | | | | |
|---|---|---|---|---|---|
| low | 75 | 49 | 29 | 11 | 3 | 2 |
| high | 91 | 73 | 55 | 19 | 6 | 4 |

**Fig. 6**

IFI16

HR = 0.41 (0.24 - 0.7)
logrank P = 6e-04

Expression
low
high

Probability

Overall survival time (months)

Number at risk
| | | | | | |
|---|---|---|---|---|---|
| low | 130 | 54 | 9 | 1 | 0 |
| high | 68 | 47 | 4 | 0 | 0 |

**Fig. 7**

IFIH1

HR = 0.28 (0.15 - 0.53)
logrank P = 3.2e-05

Expression
low
high

**Fig. 8**

IFIT1

HR = 0.58 (0.38 - 0.9)
logrank P = 0.014

Expression
low

**Fig. 9**

**IFIT3**

HR = 0.32 (0.17 - 0.57)
logrank P = 6e-05

Probability

Expression
low
high

Overall survival time (months)

Number at risk
low    134    63    9    0    0
high    64    38    4    1    0

**Fig. 10**

**LRRFIP1**

HR = 0.46 (0.28 - 0.75)
logrank P = 0.0015

Probability

Expression
low
high

Overall survival time (months)

Number at risk
low    86    59    37    12    2    0
high    80    63    47    18    7    6

**Fig. 11**

MyD88

HR = 1.39 (0.81 - 2.37)
logrank P = 0.23

Probability

Expression
low
high

Overall survival time (months)

Number at risk
| | | | | | |
|---|---|---|---|---|---|
| low | 49 | 26 | 4 | 1 | 0 |
| high | 149 | 75 | 9 | 0 | 0 |

**Fig. 12**

OAS1

HR = 0.46 (0.3 - 0.71)
logrank P = 0.00029

Probability

Expression
low
high

Overall survival time (months)

Number at risk
| | | | | | |
|---|---|---|---|---|---|
| low | 65 | 29 | 4 | 0 | 0 |
| high | 133 | 72 | 9 | 1 | 0 |

**Fig. 13**

TLR8

HR = 0.5 (0.32 - 0.78)
logrank P = 0.0021

Probability

Expression
low
high

Overall survival time (months)

Number at risk

| | | | | | |
|---|---|---|---|---|---|
| low | 101 | 46 | 6 | 1 | 0 |
| high | 97 | 55 | 7 | 0 | 0 |

**Fig. 14**

ZBP1

HR = 0.38 (0.21 - 0.69)
logrank P = 0.00086

Probability

Expression
low
high

Overall survival time (months)

Number at risk

| | | | | | |
|---|---|---|---|---|---|
| low | 111 | 75 | 49 | 13 | 1 | 1 |
| high | 55 | 47 | 35 | 17 | 8 | 5 |

**Fig. 15**

CD2

HR = 0.47 (0.3 - 0.73)
logrank P = 0.00056

Probability

Expression
low
high

Overall survival time (months)

Number at risk

| | | | | | |
|---|---|---|---|---|---|
| low | 89 | 39 | 2 | 0 | 0 |
| high | 109 | 62 | 11 | 1 | 0 |

**Fig. 16**

CD247

HR = 0.44 (0.27 - 0.71)
logrank P = 0.00052

Probability

Expression
low
high

Overall survival time (months)

Number at risk

| | | | | | |
|---|---|---|---|---|---|
| low | 114 | 50 | 6 | 1 | 0 |
| high | 84 | 51 | 7 | 0 | 0 |

**Fig. 17**

CD28

**Fig. 18**

CD3E

## Fig. 19

**CD3G**

HR = 0.34 (0.19 - 0.6)
logrank P = 0.00011

Expression
low
high

Overall survival time (months)

| Number at risk | | | | | |
|---|---|---|---|---|---|
| low | 134 | 61 | 6 | 1 | 0 |
| high | 64 | 40 | 7 | 0 | 0 |

## Fig. 20

**CD4**

HR = 0.29 (0.19 - 0.45)
logrank P = 2.4e-09

Expression
low
high

Overall survival time (months)

| Number at risk | | | | | |
|---|---|---|---|---|---|
| low | 54 | 22 | 2 | 0 | 0 |
| high | 144 | 79 | 11 | 1 | 0 |

**Fig. 21**

CSK

HR = 0.7 (0.44 - 1.13)
logrank P = 0.14

Expression
low
high

Probability

Overall survival time (months)

Number at risk

| | | | | | | |
|---|---|---|---|---|---|---|
| low | 61 | 43 | 32 | 8 | 1 | 1 |
| high | 105 | 79 | 52 | 22 | 8 | 5 |

**Fig. 22**

EZR

HR = 0.27 (0.15 - 0.5)
logrank P = 7e-06

Expression
low
high

Probability

Overall survival time (months)

Number at risk

| | | | | | | |
|---|---|---|---|---|---|---|
| low | 103 | 65 | 43 | 16 | 5 | 2 |
| high | 63 | 57 | 41 | 14 | 4 | 4 |

**Fig. 23**

FYN

**Fig. 24**

LAT

**Fig. 25**

LCK

**Fig. 26**

PAG1

**Fig. 27**

## PDE4D

HR = 0.59 (0.33 - 1.06)
logrank P = 0.072

Probability

Expression
low
high

Overall survival time (months)

Number at risk

| | | | | | | |
|---|---|---|---|---|---|---|
| low | 123 | 88 | 56 | 19 | 4 | 3 |
| high | 43 | 34 | 28 | 11 | 5 | 3 |

**Fig. 28**

## PRKACA

HR = 1.45 (0.9 - 2.32)
logrank P = 0.12

Probability

Expression
low
high

Overall survival time (months)

Number at risk

| | | | | | | |
|---|---|---|---|---|---|---|
| low | 92 | 71 | 56 | 22 | 6 | 3 |
| high | 74 | 51 | 28 | 8 | 3 | 3 |

**Fig. 29**

PRKACB

HR = 1.55 (1 - 2.41)
logrank P = 0.05

Probability

Expression
low
high

Overall survival time (months)

Number at risk
| | | | | |
|---|---|---|---|---|
| low | 97 | 54 | 10 | 1 | 0 |
| high | 101 | 47 | 3 | 0 | 0 |

**Fig. 30**

PTPRC

HR = 0.45 (0.28 - 0.73)
logrank P = 0.00081

Probability

Expression
low
high

Overall survival time (months)

Number at risk
| | | | | |
|---|---|---|---|---|
| low | 117 | 53 | 5 | 1 | 0 |
| high | 81 | 48 | 8 | 0 | 0 |

**Fig. 31**

ZAP70

**Fig. 32**

Survival

**Fig. 33**

Survival

## Fig. 34

survival_status

PDE4D7_R2>0
— <= threshold (0)
— >threshold (0)

logrank p=0.01

Survival Time [months]
training set (#93 patients)
pathology_M_stage="m0"

Number at risk
Group: <= threshold (0)

| 44 | 40 | 16 | 6 | 1 | 0 | 0 |

Group: >threshold (0)

| 49 | 40 | 17 | 3 | 1 | 1 | 0 |

## Fig. 35

Survival

SKCMAI
— <=threshold (0)
— >threshold (0)

logrank p=0.003
HR=3.5 [95% CI 1.5-7.8]

Survival Time [months]
training set (#93 patients)
pathology_M_stage="m0"

Number at risk
Group: <=threshold (0)

| 41 | 34 | 15 | 7 | 2 | 1 | 0 |

Group: >threshold (0)

| 52 | 46 | 18 | 2 | 0 | 0 | 0 |

**Fig. 36**

Survival

logrank p<0.0001
HR=3.1 [95% CI 2.2-4.3]

SKCMAI
<=threshold (0)
>thershold (0)

Survival Time [months]
training set (#367 patients)

Number at risk
Group: <=threshold (0)

| 201 | 111 | 61 | 32 | 11 | 4 | 3 | 1 | 0 |

Group: >thershold (0)

| 166 | 43 | 17 | 6 | 4 | 2 | 1 | 0 | 0 |

**Fig. 37**

Survival

logrank p=0.01
HR=3.2 [95% CI 1.6-6.4]

SKCMAI
<=threshold (0)
>threshold (0)

Survival Time [months]
testing set (#91 patients)

Number at risk
Group: <=threshold (0)

| 52 | 25 | 15 | 11 | 7 | 3 | 2 | 2 | 0 |

Group: >threshold (0)

| 39 | 8 | 0 | 0 | 0 | 0 | 0 | 0 | 0 |

## Fig. 38

Survival probability (%)

logrank p=0.0003
HR=1.7 [95% CI 1.3-2.2]

Survival Time [months]
Tumor="Melanoma"

IDR14_6.1
—— <=threshold (-0.19)
---- >threshold (-0.19)

Number at risk
Group: <=threshold (-0.19)

| 198 | 150 | 107 | 51 | 13 | 8 | 0 |

Group: >threshold (-0.19)

| 178 | 104 | 77 | 32 | 5 | 2 | 0 |

## Fig. 39

Survival probability (%)

logrank p=0.001
HR=1.6 [95% CI 1.2-2.1]

Survival Time [months]
Tumor="Melanoma"

IDR14_6.2
—— <=threshold (-2)
---- >threshold (-2)

Number at risk
Group: <=threshold (-2)

| 191 | 143 | 102 | 55 | 14 | 8 | 0 |

Group: >threshold (-2)

| 185 | 111 | 82 | 28 | 4 | 2 | 0 |

## Fig. 40

Event

Tumor="Melanoma"

IDR14_6.3
—— <=threshold (-1.6)
- - - >threshold (-1.6)

logrank p<0.0001
HR=1.9 [95% CI 1.4-2.6]

Number at risk
Group: <=threshold (-1.6)
| 222 | 166 | 126 | 61 | 14 | 8 | 0 |
Group: >threshold (-1.6)
| 154 | 88 | 58 | 22 | 4 | 2 | 0 |

## Fig. 41

Tumor="Melanoma"

TCR17_6.1>0.27
—— <=thershold (0.27)
- - - >thershold (0.27)

logrank p<0.0001
HR=2.1 [95% CI 1.6-2.8]

Number at risk
Group: <=thershold (0.27)
| 189 | 149 | 109 | 51 | 16 | 9 | 0 |
Group: >thershold (0.27)
| 187 | 105 | 75 | 32 | 2 | 1 | 0 |

## Fig. 42

TCR17_6.2>-2.5
—— <=threshold (-2.5)
--- >threshold (-2.5)

logrank p<0.0001
HR=1.8 [95% CI 1.4-2.4]

Surival Time
Tumor="Melanoma"

Number at risk
Group: <=threshold (-2.5)

| 180 | 139 | 104 | 52 | 14 | 8 | 0 |

Group: >threshold (-2.5)

| 196 | 115 | 80 | 31 | 4 | 2 | 0 |

## Fig. 43

TCR17_6.3>-2.1
—— <=threshold (-2.1)
--- >thershold (-2.1)

logrank p<0.0001
HR=2.0 [95% CI 1.5-2.7]

Survival Time [months]
Tumor="Melanoma"

Number at risk
Group: <=threshold (-2.1)

| 213 | 162 | 119 | 54 | 14 | 7 | 0 |

Group: >thershold (-2.1)

| 163 | 92 | 65 | 29 | 4 | 3 | 0 |

## Fig. 44

Number at risk
Group: <=threshold (-3.5)

|     |     |     |     |     |     |     |
|-----|-----|-----|-----|-----|-----|-----|
| 193 | 149 | 107 | 52  | 16  | 9   | 0   |

Group: >threshold (-3.5)

|     |     |     |     |     |     |     |
|-----|-----|-----|-----|-----|-----|-----|
| 183 | 105 | 77  | 31  | 2   | 1   | 0   |

## Fig. 45

Number at risk
Group: <=threshold (-2.8)

|     |     |     |     |     |     |     |
|-----|-----|-----|-----|-----|-----|-----|
| 183 | 135 | 104 | 55  | 14  | 8   | 0   |

Group: >threshold (-2.8)

|     |     |     |     |     |     |     |
|-----|-----|-----|-----|-----|-----|-----|
| 193 | 119 | 80  | 28  | 4   | 2   | 0   |

## Fig. 46

Survival Time [months]
Tumor="Melanoma"

SKCMAI_6.3
—— <=threshold (-2.3)
--- >threshold (-2.3)

logrank p<0.0001
HR=2.0 [95% CI 1.5-2.6]

Number at risk
Group: <=threshold (-2.3)

| 171 | 136 | 105 | 53 | 13 | 8 | 0 |

Group: >threshold (-2.3)

| 205 | 118 | 79 | 30 | 5 | 2 | 0 |

**Fig. 47**

Survival

Number at risk
Group: <=threshold (-3.3)
|  |  |  |  |  |  |  |
|---|---|---|---|---|---|---|
| 74 | 48 | 27 | 13 | 6 | 3 | 0 |

Group: >threshold (-3.3)
|  |  |  |  |  |  |  |
|---|---|---|---|---|---|---|
| 62 | 45 | 35 | 17 | 3 | 3 | 0 |

**Fig. 48**

Survival

Number at risk
Group: <=threshold (-3.3)
|  |  |  |  |  |  |  |
|---|---|---|---|---|---|---|
| 77 | 54 | 43 | 17 | 0 | 0 | 0 |

Group: >threshold (-3.3)
|  |  |  |  |  |  |  |
|---|---|---|---|---|---|---|
| 83 | 51 | 37 | 23 | 4 | 2 | 0 |

## Fig. 49

Survival

Survival probability (%)

logrank p=0.4

Survival Time [months]
Tumor="Melanoma"; SKCMAI_low

SKCMAI (CoxReg mean)
— SKCMAI <=-3.3 & PEMBRO
— SKCMAI <=-3.3 & NIVO

Number at risk
Group: SKCMAI <=-3.3 & PEMBRO

| 74 | 48 | 27 | 13 | 6 | 3 | 0 |

Group: SKCMAI <=-3.3 & NIVO

| 77 | 54 | 43 | 17 | 0 | 0 | 0 |

## Fig. 50

Survival

Survival probability (%)

logrank p=0.04
HR=1.6 [95% CI 1.0-2.5]

Survival Time [months]
Tumor="Melanoma"; SKCMAI_high

SKCMAI (CoxReg mean)
— SKCMAI>-3.3 & PEMBRO
— SKCMAI>-3.3 & NIVO

Number at risk
Group: SKCMAI>-3.3 & PEMBRO

| 62 | 45 | 35 | 17 | 3 | 3 | 0 |

Group: SKCMAI>-3.3 & NIVO

| 83 | 51 | 37 | 23 | 4 | 2 | 0 |

**Fig. 51**

Survival

Survival probability (%)

SKCMAI (CoxReg mean)
— IPI
— IPI+NIVO
— IPI+PEMBRO
— NIVO
— PEMBRO

logrank p=0.6

Survival Time [months]
Tumor="Melanoma", SKCMAI_low

Number at risk
Group: IPI

| | | | | | | |
|---|---|---|---|---|---|---|
| 7 | 4 | 3 | 3 | 0 | 0 | 0 |

Group: IPI+NIVO

| | | | | | | |
|---|---|---|---|---|---|---|
| 3 | 2 | 0 | 0 | 0 | 0 | 0 |

Group: IPI+PEMBRO

| | | | | | | |
|---|---|---|---|---|---|---|
| 6 | 6 | 2 | 0 | 0 | 0 | 0 |

Group: NIVO

| | | | | | | |
|---|---|---|---|---|---|---|
| 77 | 54 | 43 | 17 | 0 | 0 | 0 |

Group: PEMBRO

| | | | | | | |
|---|---|---|---|---|---|---|
| 74 | 48 | 27 | 13 | 6 | 3 | 0 |

**Fig. 52**

Survival

Survival probability (%)

SKCMAI (CoxReg mean)
— IPI
— IPI+NIVO
— IPI+PEMBRO
— NIVO
— PEMBRO

logrank p=0.001

Survival Time [months]
Tumor="Melanoma", SKCMAI_high

Number at risk
Group: IPI

| | | | | | | |
|---|---|---|---|---|---|---|
| 35 | 19 | 17 | 9 | 5 | 2 | 0 |

Group: IPI+NIVO

| | | | | | | |
|---|---|---|---|---|---|---|
| 5 | 2 | 1 | 1 | 0 | 0 | 0 |

Group: IPI+PEMBRO

| | | | | | | |
|---|---|---|---|---|---|---|
| 24 | 23 | 19 | 0 | 0 | 0 | 0 |

Group: NIVO

| | | | | | | |
|---|---|---|---|---|---|---|
| 83 | 51 | 37 | 23 | 4 | 2 | 0 |

Group: PEMBRO

| | | | | | | |
|---|---|---|---|---|---|---|
| 62 | 45 | 35 | 17 | 3 | 3 | 0 |

**EUROPEAN SEARCH REPORT**

Application Number

EP 24 20 4475

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/218649 A1 (SAENGER YVONNE [US] ET AL) 6 August 2015 (2015-08-06) * the whole document * * para. 98, 100-103, 122, 171, 175, 306-308, 373, Fig. 4-7 * | 1-15 | INV. C12Q1/6886 |
| Y | WO 2021/110927 A1 (SERVICIO ANDALUZ DE SALUD [ES]; UNIV MALAGA [ES]) 10 June 2021 (2021-06-10) * the whole document * * p. 2, ll. 9-12, Fig. 1 * | 1-15 | |
| Y | WO 2024/112609 A1 (MERCK SHARP & DOHME LLC [US]) 30 May 2024 (2024-05-30) * the whole document * * p. 1, ll. 15-22, Tables 1-2 * | 1-15 | |
| Y | WAGNER NIKOLAUS B ET AL: "S100B and LDH as early prognostic markers for response and overall survival in melanoma patients treated with anti-PD-1 or combined anti-PD-1 plus anti-CTLA-4 antibodies", BRITISH JOURNAL OF CANCER, NATURE PUBLISHING GROUP UK, LONDON, vol. 119, no. 3, 28 June 2018 (2018-06-28) , pages 339-346, XP036683718, ISSN: 0007-0920, DOI: 10.1038/S41416-018-0167-X [retrieved on 2018-06-28] * the whole document * * abstract; p. 1, col. 1, last para. * | 1-15 | TECHNICAL FIELDS SEARCHED (IPC) C12Q |

The present search report has been drawn up for all claims

| Place of search | Date of completion of the search | Examiner |
|---|---|---|
| The Hague | 13 March 2025 | Sauer, Tincuta |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

**Application Number**

**EP 24 20 4475**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

Application Number

EP 24 20 4475

The Search Division considers that the present European patent application does not comply with the requirements of unity of invention and relates to several inventions or groups of inventions, namely:

1. claims: 1-15(partially)

   Pembroluzimab or an equivalent thereof for use in the treatment of a melanoma in a subject, the use comprising:determining or receiving the result of a determination of one or more gene expression levels and determining the prediction of the outcome based on one or more gene expression levels, wherein the outcome is a poor outcome or a good outcome; Method as defined in present claim 5; Products as defined in claims 13-14; Use of a kit as defined in claim 15; wherein said one or more gene comprise CD4.

   ---

2-24. claims: 1-15(partially)

   Pembroluzimab or an equivalent thereof for use in the treatment of a melanoma in a subject, the use comprising:determining or receiving the result of a determination of one or more gene expression levels and determining the prediction of the outcome based on one or more gene expression levels, wherein the outcome is a poor outcome or a good outcome; Method as defined in present claim 5; Products as defined in claims 13-14; Use of a kit as defined in claim 15; wherein said one or more gene comprise each further gene listed in claim 1.

   ---

## ANNEX TO THE EUROPEAN SEARCH REPORT
## ON EUROPEAN PATENT APPLICATION NO.

EP 24 20 4475

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2025

| Patent document cited in search report | Publication date | Patent family member(s) | | Publication date |
|---|---|---|---|---|
| US 2015218649 A1 | 06-08-2015 | AU | 2013296233 A1 | 19-03-2015 |
| | | CA | 2918608 A1 | 06-02-2014 |
| | | US | 2015218649 A1 | 06-08-2015 |
| | | WO | 2014022826 A2 | 06-02-2014 |
| WO 2021110927 A1 | 10-06-2021 | EP | 4069869 A1 | 12-10-2022 |
| | | WO | 2021110927 A1 | 10-06-2021 |
| WO 2024112609 A1 | 30-05-2024 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022043299 A1
- WO 2021175986 A1

**Non-patent literature cited in the description**

- **SAMBROOK et al.** Molecular Cloning. A Laboratory Manual. Cold Spring Harbor Laboratory Press, 1989
- **AUSUBEL et al.** Current Protocols in Molecular Biology. John Wiley & Sons, 1987
- Methods in Enzymology. Academic Press
- Computational Molecular Biology. Oxford University Press, 1988
- Biocomputing: Informatics And Genome Projects. Academic Press, 1993
- Computer Analysis Of Sequence Data. Humana Press, 1994
- Sequence Analysis In Molecular Biology. Academic Press, 1987
- Sequence Analysis Primer. M Stockton Press, 1991
- **CARILLO, H.** ; **LIPTON, D.** *SIAM J. Applied Math*, 1988, vol. 48, 1073
- Guide To Huge Computers. Academic Press, 1994
- **CARILLO, H.** ; **LIPTON, D.** *Siam J. Applied Math*, 1988, vol. 48, 1073
- **DEVEREUX, J. et al.** *Nucleic Acids Research*, 1984, vol. 12 (1), 387
- **ATSCHUL, S. F. et al.** *J. Molec. Biol.*, 1990, vol. 215, 403
- **AKBANI, REHAN et al.** Genomic Classification of Cutaneous Melanoma. *Cell*, vol. 161 (7), 1681-1696
- **GASSER S. et al.** Sensing of dangerous DNA. *Mechanisms of Aging and Development*, 2017, vol. 165, 33-46
- **MOSENDEN R.** ; **TASKEN K.** Cyclic AMP-mediated immune regulation - Overview of mechanisms of action in T-cells. *Cell Signal*, 2011, vol. 23 (6), 1009-1016
- **TASKEN K.** ; **RUPPELT A.** Negative regulation of T-cell receptor activation by the cAMP-PKA-Csk signaling pathway in T-cell lipid rafts. *Front Biosci*, 2006, vol. 11, 2929-2939
- **ABRAHAMSEN H. et al.** TCR- and CD28-mediated recruitment of phosphodiesterase 4 to lipid rafts potentiates TCR signaling. *J Immunol*, 2004, vol. 173, 4847-4848